# NEW EUROPEAN PATENT SPECIFICATION

(11) **EP 1 341 909 B2**
(45) Date of publication and mention of the opposition decision: **11.04.2012**
(45) Mention of the grant of the patent: 18.05.2005
(21) Application number: 01985402.5
(22) Date of filing: 12.12.2001
(51) Int. Cl.: C12N 15/10, C12Q 1/68

(54) **A METHOD FOR IN VITRO MOLECULAR EVOLUTION OF PROTEIN FUNCTION**
EINE METHODE ZUR IN VITRO MOLEKULAREN EVOLUTION DER PROTEINFUNKTION
PROCEDE DESTINE A L'EVOLUTION MOLECULAIRE IN VITRO D'UNE FONCTION PROTEINIQUE

(30) Priority: 12.12.2000 GB 0030252; 12.12.2000 US 734801
(43) Date of publication of application: 10.09.2003
(73) Proprietor: Alligator Bioscience AB, 223 70 Lund (SE)
(72) Inventor: CARLSSON, Roland, S-226 54 Lund (SE); FUREBRING, Christina, S-224 72 Lund (SE); MALMBORG HAGER, Ann-Christin, S-252 50 Helsingborg (SE); BORREBAECK, Carl, S-245 62 Hjarup (SE)
(74) Representative: Thomas, Philip John Duval
(86) International application number: PCT/EP2001/014744
(87) International publication number: WO 2002/048351

(56) References cited:
- WO-A-00/72013
- WO-A-98/58080
- WO-A1-97/20078
- WO-A1-98/58080
- US-A- 6 143 527
- KIKUCHI M ET AL: "An effective family shuffling method using single-stranded DNA" GENE, ELSEVIER BIOMEDICAL PRESS. AMSTERDAM, NL, vol. 243, no. 1-2, February 2000 (2000-02), pages 133-137, XP004187682 ISSN: 0378-1119 cited in the application
- KIKUCHI ET AL: 'Novel family shuffling methods for the invitro evolution of enzymes' GENE vol. 236, 01 January 1999, pages 159 - 167
- BROWN T.A.: 'Molecular Biology labFax, I:Recombinant DNA' ACADEMIC PRESS 01 January 1998,
- ZHA ET AL: 'Methods in Molecular Biology, Directed Evolution Library Creation. Methods and Protocols.' HUMAN PRESS INC. F.H.ARNOLD & G.GEORGIOU 01 January 2003,

## Description

The present invention relates to a method for in vitro molecular evolution of protein function, in particular by shuffling of single stranded DNA segments obtained using a nuclease.

Protein function can be modified and improved *in vitro* by a variety of methods, including site directed mutagenesis (Alber *et al*., Nature, 5; 330(6143):41-46, 1987) combinatorial cloning (Huse *et al*., Science, 246:1275-1281, 1989; Marks *et al*., Biotechnology, 10: 779-783, 1992) and random mutagenesis combined with appropriate selection systems (Barbas *et al*., PNAS. USA, 89: 4457-4461, 1992).

The method of random mutagenesis together with selection has been used in a number of cases to improve protein function and two different strategies exist. Firstly, randomisation of the entire gene sequence in combination with the selection of a variant (mutant) protein with the desired characteristics, followed by a new round of random mutagenesis and selection. This method can then be repeated until a protein variant is found which is considered optimal (Schier R. *et al*., J. Mol. Biol. 1996 263 (4): 551-567). Here, the traditional route to introduce mutations is by error prone PCR (Leung *et al*., Technique, 1: 11-15, 1989) with a mutation rate of approximately 0.7%. Secondly, defined regions of the gene can be mutagenised with degenerate primers, which allows for mutation rates up to 100% (Griffiths *et al*., EMBO. J, 13: 3245-3260, 1994; Yang *et al*., J. Mol.

Biol. 254: 392-403, 1995). The higher the mutation rate used, the more limited the region of the gene that can be subjected to mutations.

Random mutation has been used extensively in the field of antibody engineering. *In vivo* formed antibody genes can be cloned *in vitro* (Larrick *et al*., Biochem. Biophys. Res. Commun. 160: 1250-1256, 1989) and random combinations of the genes encoding the variable heavy and light genes can be subjected to selection (Marks *et al*., Biotechnology, 10: 779-783, 1992). Functional antibody fragments selected can be further improved using random mutagenesis and additional rounds of selections (Schier R. *et al*., J. Mol. Biol. 1996 263 (4): 551-567).

The strategy of random mutagenesis is followed by selection. Variants with interesting characteristics can be selected and the mutated DNA regions from different variants, each with interesting characteristics, are combined into one coding sequence (Yang *et al*., J. Mol. Biol. 254: 392-403, 1995). This is a multi-step sequential process, and potential synergistic effects of different mutations in different regions can be lost, since they are not subjected to selection in combination. Thus, these two strategies do not include simultaneous mutagenesis of defined regions and selection of a combination of these regions. Another process involves combinatorial pairing of genes which can be used to improve e.g. antibody affinity (Marks *et al*., Biotechnology, 10: 779-783, 1992). Here, the three CDR-regions in each variable gene are fixed and this technology does not allow for shuffling of individual gene segments in the gene for the variable domain, for example, including the CDR regions, between clones.

The concept of DNA shuffling (Stemmer, Nature 370: 389-391, 1994) utilises random fragmentation of DNA and assembly of fragments into a functional coding sequence. In this process it is possible to introduce chemically synthesised DNA sequences and in this way target variation to defined places in the gene which DNA sequence is known (Crameri *et al*., Biotechniques, 18: 194-196, 1995). Stemmer and coworkers developed this *in vitro* method, which reassemble the normally occurring evolution process of protein in nature. The DNA shuffling generates diversity by recombination, combining useful mutations from individual genes. It has been used successfully for artificial evolution of different proteins, e.g. enzymes and cytokines (Chang *et al*. Nature Biotech. 17, 793-797, 1999; Zhang *et al*. Proc. Natl. Acad. Sci. USA 94, 4504-4509,1997; Christians *et al*. Nature Biotech. 17, 259-264, 1999). The genes are randomly fragmented using DNase I and then reassembled by recombination with each other. The starting material can be either a single gene (first randomly mutated using error-prone PCR) or naturally occurring homologous sequences so called family shuffling. DNase I hydrolyses DNA preferentially at sites adjacent to pyrimidine nucleotides, therefore it is a suitable choice for random fragmentation of DNA. However, the activity is dependent on Mg or Mn ions, Mg ions restrict the fragment size to 50bp, while the Mn ions will give fragment sizes less than 50bp. Therefore, in order to have all possible sizes for recombination the gene in question needs to be treated at least twice with DNase I in the presence of either of the two different ions, followed by removal of these very same ions.

In theory, it is possible to shuffle DNA between any clones. However, if the resulting shuffled gene is to be functional with respect to expression and activity, the clones to be shuffled have preferably to be related or even identical with the exception of a low level of random mutations. DNA shuffling between genetically different clones will generally produce non-functional genes. However, it has been proven by the methodology of ITCHY that interspecies fusion libraries can be created between fragments of the *E. coli* and human glycinamide ribonucleotide transformylase genes, which have only 50% identity on the DNA level (Ostermeier *et al*., Nat Biotechnol 17, 1205-9, 1999).

A successful recombination of two different genes requires formation of hetero-duplex molecules. In some cases the family shuffling almost only form homo-duplexes resulting in a low frequency of recombination. This problem has been addressed by using DNase I-digested single-stranded DNA (Kikuchi *et al*. Gene 243,133-137 2000).

Single-stranded DNA can be obtained in essentially two different ways. Firstly, by the use of biotinylated primers in the PCR reactions in combination with e.g. Dynabeads (Dynal, Norway) or AffiniTip Streptavidin Capture Micro-columns (Genosys Biotechnologies Inc., The Woodlands, USA). Secondly, by utilising bacteriophage that are able to pack single-stranded DNA (Viruses and Related Entities in Modem Microbiology, Principles and Applications pp.171-192, Ed. E.A. Birge, Wm. C. Brown Publishers1992; Sambrook *et al*. Molecular Cloning, A laboratory manual 2^{nd} edition. Cold Spring Habor Laboratory Press, 1989).

Selection of enzymes with altered and improved properties are often based on the actual function of the enzyme. For example increased thermostability of an enzyme can be selected for by incubating transformed colonies at temperatures that cause inactivation of wild type enzyme and improved β-glucosidase activity can be identified by using PNPG as the substrate (Arrizubieta *et al* J Biol Chem Jun 27, 2000).

Selection of functional proteins from molecular libraries has been revolutionised by the development of the phage display technology (Parmley *et al*., Gene, 73: 305-391 1988; McCafferty *et al*., Nature, 348: 552-554, 1990; Barbas *et al*., PNAS. USA, 88: 7978-7982, 1991). Here, the phenotype (protein) is directly linked to its corresponding genotype (DNA) and this allows for directly cloning of the genetic material which can then be subjected to further modifications in order to improve protein function. Phage display has been used to clone functional binders from a variety of molecular libraries with up to 10¹¹ transformants in size (Griffiths *et al*., EMBO. J. 13: 3245-3260, 1994). Thus, phage display can be used to directly clone functional binders from molecular libraries, and can also be used to improve further the clones originally selected. Other types of viruses that have been used for surface expression of protein libraries and selections thereof are baculovirus (Boublik *et al* Biotechnol 13:1079-1084. 1995; Mottershead *et al* Biochem Biophys Res Com 238:717-722, 1997; Grabherr *et al* Biotechniques 22:730-735, 1997) and retrovirus (Buchholz *et al* Nature Biotechnol 16:951-954, 1998).

Selection of functional proteins from molecular libraries can also be performed by cell surface display. Also here, the phenotype is directly linked to its corresponding genotype. Bacterial cell surface display has been used for e.g. screening of improved variants of carboxymethyl cellulase (CMCase) (Kim *et al* Appl Environ Microbiol 66:788-93, 2000). Other cells that can be used for this purpose are yeast cells (Boder and Wittrup Nat. Biotechnol 15:553-557, 1997), COS cells (Higuchi *et al* J Immunol Meth 202:193-204, 1997), and insect cells (Granzerio *et al* J Immunol Meth 203:131-139, 1997; Ernst *et al* Nucleic Acids Res 26:1718-1723, 1998).

Random combination of DNA from different mutated clones in combination with selection of desired function is a more efficient way to search through sequence space as compared to sequential selection and combination of selected clones.

This invention seeks to provide improved methods for *in vitro* protein evolution. In particular, the invention aims to provide more efficient recombination and shuffling methods, which will give rise to more altered molecules and thereby improve the probability of finding molecules with desirable properties.

According to one aspect of the present invention, there is provided a method for generating a polynucleotide sequence or population of sequences from parent single stranded polynucleotide sequences encoding one or more protein motifs, comprising the steps of
a) providing single stranded DNA constituting plus and minus strands of parent polynucleotide sequences;
b) digesting the single stranded polynucleotide sequences with an exonuclease to generate populations of single stranded fragments;
c) contacting said fragments generated from the plus strands with fragments generated from the minus strands and optionally, adding primer sequences that anneal to the 3'and 5'ends of at least one of the parent polynucleotides under annealing conditions;
d) amplifying the fragments that anneal to each other to generate at least one polynucleotide sequence encoding one or more protein motifs having altered characteristics as compared to the one or more protein motifs encoded by said parent polynucleotides.

Therefore, typically, there is provided a method of combining polynucleotide fragments to generate a polynucleotide sequence or population of sequences of desired characteristics, which method comprises the steps of:
a) digesting a linear parent single-stranded polynucleotide encoding one or more protein motifs with a nuclease other than DNase I to generate a population of single-stranded fragments of varying lengths;
b) assembling a polynucleotide sequence from the sequences derived from step (a).

Preferably the method further comprises the step of (c) expressing the resulting protein encoded by the assembled polynucleotide sequence and d) screening the protein for desired characteristics.

By controlling the reaction time of the nuclease the size of the polynucleotide fragments may be controlled. Determining the lengths of the polynucleotide fragments in this way avoids the necessity of having to provide a further step such as purifying the fragments of desired length from a gel.

In order to generate a polynucleotide sequence of desired characteristics the parent polynucleotide encoding one or more protein motifs may be subjected to mutagenesis to create a plurality of differently mutated derivatives thereof. Likewise, a parent polynucleotide may be obtained already encoding a plurality of variant protein motifs of unknown sequence.

Random mutation can be accomplished by any conventional method as described above, but a suitable method is error-prone PCR.

It is preferable to use PCR technology to assemble the single-stranded polynucleotide fragments into a double-stranded polynucleotide sequence.

The polynucleotide sequence is preferably DNA although RNA may be used. For simplicity the term polynucleotide will now be used in the following text in relation to DNA but it will be appreciated that the present invention is applicable to both RNA and DNA.

Preferably, any exonuclease that digests polynucleotide from the 5' prime end to the 3' prime, from the 3' to the 5' end or from both the 3' and the 5' ends may be used. Examples of a suitable exonuclease which may be used in accordance with the present invention include BAL31, exonuclease VII and exonuclease Rec J_{f}.

Preferably, the exonuclease is BAL31.

Using BAL31 nuclease in the DNA shuffling process of the invention provides a fast, easy and controllable system. This enzyme can give all sizes of gene fragments and the activity of the enzyme can be easily controlled by stopping the digestion at various time points. BAL 31 is predominately a 3' prime exonuclease that removes mononucleotides from both 3' termini of the two strands of a linear DNA. BAL 31 is also an endonuclease; thus the single-stranded DNA generated by the 3' prime exonuclease activity is degraded by the endonuclease. The 3' prime exonuclease activity of the enzyme works about 20-fold more efficiently than the endonuclease. The enzyme concentrations are therefore important for the obtained DNA fragments. High concentration of enzyme favours blunt-ended DNA whereas at low concentrations the single-stranded DNA termini may be very long. BAL 31 consists of two kinetically distinct forms of the enzyme, a fast (F) and a slow (S) form. The S form is a proteolytic degradation product of the F form. Furthermore, BAL 31 works asynchronously, generating a population of DNA molecules whose termini have been resected to various extents and whose single-stranded tails vary in length. Both forms also act on ssDNA in an exonucleolytic fashion in a highly processive manner. The direction of attack is from the 5' end, in contrast to the mode of digestion of duplex DNA. It has been suggested that the nuclease molecules initially are non-productively bound away from the 5'ends and undergo facilitated diffusion to yield productive (terminally bound) enzyme-substrate complexes (Lu T and Gray jr. HB Biochimica et Biophysica Acta 1995, vol. 1251, p125-138). The enzyme uses Ca²⁺ as a cofactor which can be bound in complex with EGTA (Ethylene Glycol bis (β-amino ethyl Ether) N,N,N',N'-tetra acetic acid). Linear DNA sequences are digested with BAL31 and the reaction stopped at different time points by the addition of EGTA.

The individual digested fragments are purified, mixed and reassembled with PCR technology. The assembled (reconstituted) gene may then be cloned into an expression vector for expressing the protein. The protein may then be analyzed for improved characteristics.

The method of the present invention provides several advantages over known shuffling techniques, including increased rates of recombination and control of fragment size.

The method of the present invention produces a set of progressively shortened DNA fragments for each time point a DNA sample is taken from the BAL31 treatment. The DNA samples may be collected and pooled together or, optionally, individual samples may be chosen and used in the method. Thus the present invention allows a selection of what DNA samples are to be used in the recombination system and thereby offers a further degree of control.

The method of the present invention may be carried out on any polynucleotide which codes for a particular product for example any protein having binding or catalytical properties e.g. antibodies or parts of antibodies, enzymes or receptors. Further, any polynucleotide that has a function that may be altered for example catalytical RNA may be shuffled in accordance with the present invention. It is preferable that the parent polynucleotide encoding one or more protein motif is at least 12 nucleotides in length, more preferably at least 20 nucleotides in length, even more preferably more than 50 nucleotides in length. Polynucleotides being at least 100 nucleotides in length or even at least 200 nucleotides in length may be used. Where parent polynucleotides are used that encode large proteins such as enzymes or antibodies, these may be many hundreds or thousands of bases in length. The present invention may be carried out on any size of parent polynucleotide.

The present invention also provides polynucleotide sequences generated by the method described above having desired characteristics. These sequences may be used for generating gene therapy vectors and replication-defective gene therapy constructs or vaccination vectors for DNA-based vaccinations. Further, the polynucleotide sequences may be used as research tools.

The present invention also provides a polynucleotide library of sequences generated by the method described above from which a polynucleotide may be selected which encodes a protein having the desired characteristics. It is preferable that the polynucleotide library is a DNA or cDNA library.

The present inventions also provides proteins such as enzymes, antibodies, and receptors having characteristics different to that of the wild type produced by the method described above. These proteins may be used individually or within a pharmaceutically acceptable carrier as vaccines or medicaments for therapy, for example, as immunogens, antigens or otherwise in obtaining specific antibodies. They may also be used as research tools.

The desired characteristics of a polynucleotide generated by the present invention or a protein encoded by a polynucleotide generated by the present invention may be any variation or alteration in the normal activity of the wild type (parent) polynucleotide or the polypeptide, protein or protein motifs it encodes. For example, it may be desirable to reduce or increase the catalytic activity of an enzyme, or improve or reduce the binding specificity of an antibody. Further, if the protein, or polynucleotide is an immunogen, it may be desirable to reduce or increase its ability to obtain specific antibodies against it. The parent polynucleotide preferably encodes one or more protein motifs. These are defined by regions of polynucleotide sequence, that encode polypeptide sequence having or potentially having characteristic protein function. For example, a protein motif may define a portion of a whole protein, i.e. an epitope or a cleavage site or a catalytic site etc. However, within the scope of the present invention, an expressed protein motif does not have to display activity, or be "correctly" folded.

It may be desirable to modify a protein so as to alter the conformation of certain epitopes, thereby improving its antigenicity and/or reducing cross-reactivity. For example, should such a protein be used as an antigen, the modification may reduce any cross-reaction of raised antibodies with similar proteins.

Although the term "enzyme" is used, this is to be interpreted as also including any polypeptide having enzyme-like activity, i.e. a catalytic function. For example, polypeptides being part of an enzyme may still possess catalytic function. Furthermore, proteins such as interferons and cytokines are included. Likewise, the term "antibody" should be construed as covering any binding substance having a binding domain with the required specificity. This includes antibody fragments, derivatives, functional equivalents and homologues of antibodies, including synthetic molecules and molecules whose shape mimics that of an antibody enabling it to bind an antigen or epitope. Examples of antibody fragments, capable of binding an antigen or other binding partner are Fab fragment consisting of the VL, VH, Cl and CHl domains, the Fd fragment consisting of the VH and CHl domains; the Fv fragment consisting of the VL and VH domains of a single arm of an antibody; the dAb fragment which consists of a VH domain; isolated CDR regions and F(ab')2 fragments, a bivalent fragment including two Fab fragments linked by a disulphide bridge at the hinge region. Single chain Fv fragments are also included.

In order to obtain expression of the generated polynucleotide sequence, the sequence may be incorporated in a vector having control sequences operably linked to the polynucleotide sequence to control its expression. The vectors may include other sequences such as promoters or enhancers to drive the expression of the inserted polynucleotide sequence, further polynucleotide sequences so that the protein encoded for by the polynucleotide is produced as a fusion and/or nucleic acid encoding secretion signals so that the protein produced in the host cell is secreted from the cell. The protein encoded for by the polynucleotide sequence can then be obtained by transforming the vectors into host cells in which the vector is functional, culturing the host cells so that the protein is produced and recovering the protein from the host cells or the surrounding medium. Prokaryotic and eukaryotic cells are used for this purpose in the art, including strains of *E. coli*, yeast, and eukaryotic cells such as COS or CHO cells. The choice of host cell can be used to control the properties of the protein expressed in those cells, e.g. controlling where the protein is deposited in the host cells or affecting properties such as its glycosylation.

The protein encoded by the polynucleotide sequence may be expressed by methods well known in the art. Conveniently, expression may be achieved by growing a host cell in culture, containing such a vector, under appropriate conditions which cause or allow expression of the protein.

Systems for cloning and expression of a protein in a variety of different host cells are well known. Suitable host cells include bacteria, eukaryotic cells such as mammalian and yeast, and baculovirus systems. Also, utilising the retrovirus system for cloning and expression is a good alternative, since this virus can be used together with a number of cell types. Mammalian cell lines available in the art for expression of a heterologous polypeptide include Chinese hamster ovary cells, HeLa cells, baby hamster kidney cells, COS cells and many others. A common, preferred bacterial host is *E. coli*.

Suitable vectors can be chosen or constructed, containing appropriate regulatory sequences, including promoter sequences, terminator fragments, polyadenylation sequences, enhancer sequences, marker genes and other sequences as appropriate. Vectors may be plasmids, viral e.g. phage, or phagemid, as appropriate. For further details see, for example, Molecular Cloning: a Laboratory Manual: 2nd edition, Sambrook *et al*., 1989, Cold Spring Harbor Laboratory Press. Many known techniques and protocols for manipulation of polynucleotide sequences, for example in preparation of polynucleotide constructs, mutagenesis, sequencing, introduction of DNA into cells and gene expression, and analysis of proteins, are described in detail in Current Protocols in Molecular Biology, Ausubel *et al*. eds., John Wiley & Sons, 1992.

The system can be used for the creation of DNA libraries comprising variable sequences which can be screened for the desired protein function in a number of ways. Enzyme function can be screened for with methods specific for the actual enzyme function e.g. CMCase activity, β-glucosidase activity and also thermostability. Furthermore, phage display and cell surface display may be used for screening for enzyme function (Crameri A. *et al*., Nature 1998 15; 391 (6664):288-291; Zhang J. H. *et al*., PNAS. USA 1997 94 (9): 4504-4509; Warren M.S. *et al*., Biochemistry 1996, 9; 35(27): 8855-8862; Kim *et al*., Appl Environ Microbiol 66:788-93, 2000) as well as for altered binding properties of *e.g.* antibodies (Griffith *et al*., EMBO J. 113: 3245-3260, 1994).

A protein provided by the present invention may be used in screening for molecules which affect or modulate its activity or function. Such molecules may be useful in a therapeutic (possibly including prophylactic) context.

The present invention also provides vectors comprising polynucleotide sequences generated by the method described above.

The present inventions also provides compositions comprising either polynucleotide sequences, vectors comprising the polynucleotide sequences or proteins generated by the method described above and a pharmaceutically acceptable carrier or a carrier suitable for research purposes.

The present invention also provides a method comprising, following the identification of the polynucleotide or polypeptide having desired characteristics by the method described above, the manufacture of that polypeptide or polynucleotide in whole or in part, optionally in conjunction with additional polypeptides or polynucleotides.

Following the identification of a polynucleotide or polypeptide having desired characteristics, these can then be manufactured to provide greater numbers by well known techniques such as PCR, cloning and expression within a host cell.

The resulting polypeptides or polynucleotides may be used in the preparation of industrial enzymes, e.g. laundry detergent enzymes where an increased activity is preferred at lower temperatures. Alternatively, the manufactured polynucleotide or polypeptide may be used as a research tool, i.e. antibodies may be used in immunoassays, and polynucleotides may be used as hybridization probes or primers. Alternatively, the resulting polypeptides or polynucleotides may be used in the preparation of medicaments for diagnostic use, pharmaceutical use, therapy etc. as discussed as follows.

The polypeptides or polynucleotides generated by the method of the invention and identified as having desirable characteristics can be formulated in pharmaceutical compositions. These compositions may comprise, in addition to one of the above substances, a pharmaceutically acceptable excipient, carrier, buffer, stabilizer or other materials well known to those skilled in the art. Such materials should be non-toxic and should not interfere with the efficacy of the active ingredient. The precise nature of the carrier or other material may depend on the route of administration, e.g. oral, intravenous, cutaneous or subcutaneous, nasal, intramuscular, intraperitoneal routes.

Pharmaceutical compositions for oral administration may be in tablet, capsule, powder or liquid form. A tablet may include a solid carrier such as gelatin or an adjuvant. Liquid pharmaceutical compositions generally include a liquid carrier such as water, petroleum, animal or vegetable oils, mineral oil or synthetic oil. Physiological saline solution, dextrose or other saccharide solution or glycols such as ethylene glycol, propylene glycol or polyethylene glycol may be included.

For intravenous, cutaneous or subcutaneous injection, or injection at the site of affliction, the active ingredient will be in the form of a parenterally acceptable aqueous solution which is pyrogen-free and has suitable pH, isotonicity and stability. Those of relevant skill in the art are well able to prepare suitable solutions using, for example, isotonic vehicles such as Sodium Chloride Injection, Ringer's Injection, Lactated Ringer's Injection. Preservatives, stabilizers, buffers, antioxidants and/or other additives may be included, as required.

Whether it is a polypeptide, e.g. an antibody or fragment thereof, an enzyme, a polynucleotide or nucleic acid molecule, identified following generation by the present invention that is to be given to an individual, administration is preferably in a "prophylactically effective amount" or a "therapeutically effective amount" (as the case may be, although prophylaxis may be considered therapy), this being sufficient to show benefit to the individual. The actual amount administered, and rate and time-course of administration, will depend on the nature and severity of what is being treated. Prescription of treatment, e.g. decisions on dosage etc, is within the responsibility of general practitioners and other medical doctors, and typically takes account of the disorder to be treated, the condition of the individual patient, the site of delivery, the method of administration and other factors known to practitioners. Examples of the techniques and protocols mentioned above can be found in Remington's Pharmaceutical Sciences, 16th edition, Osol, A. (ed), 1980.

Alternatively, targeting therapies may be used to deliver the active agent more specifically to certain types of cell, by the use of targeting systems such as antibody or cell specific ligands. Targeting may be desirable for a variety of reasons; for example if the agent is unacceptably toxic, or if it would otherwise require too high a dosage, or if it would not otherwise be able to enter the target cells.

Instead of administering these agents directly, they could be produced in the target cells by expression from an encoding gene introduced into the cells, e.g. in a viral vector (a variant of the VDEPT technique i.e. the activating agent, e.g. an enzyme, is produced in a vector by expression from encoding DNA in a viral vector). The vector could be targeted to the specific cells to be treated, or it could contain regulatory elements which are switched on more or less selectively by the target cells.

Alternatively, the agent could be administered in a precursor form, for conversion to the active form by an activating agent produced in, or targeted to, the cells to be treated. This type of approach is sometimes known as ADEPT or VDEPT; the former involving targeting the activating agent to the cells by conjugation to a cell-specific antibody, while the latter involves producing the activating agent, e.g. an enzyme, in a vector by expression from encoding DNA in a viral vector (see for example, EP-A-415731 and WO 90/07936).

A composition may be administered alone or in combination with other treatments, either simultaneously or sequentially dependent upon the condition to be treated.

As a further alternative, the polynucleotide identified as having desirable characteristics following generation by the method of the present invention could be used in a method of gene therapy, to treat a patient who is unable to synthesize the active polypeptide encoded by the polynucleotide or unable to synthesize it at the normal level, thereby providing the effect provided by the corresponding wild-type protein.

Vectors such as viral vectors have been used in the prior art to introduce polynucleotides into a wide variety of different target cells. Typically the vectors are exposed to the target cells so that transfection can take place in a sufficient proportion of the cells to provide a useful therapeutic or prophylactic effect from the expression of the desired polypeptide. The transfected nucleic acid may be permanently incorporated into the genome of each of the targeted tumour cells, providing long lasting effect, or alternatively the treatment may have to be repeated periodically.

A variety of vectors, both viral vectors and plasmid vectors, are known in the art, see US Patent No. 5,252,479 and WO 93/07282. In particular, a number of viruses have been used as gene transfer vectors, including papovaviruses, such as SV40, vaccinia virus, herpes viruses, including HSV and EBV, and retroviruses. Many gene therapy protocols in the prior art have used disabled murine retroviruses.

As an alternative to the use of viral vectors other known methods of introducing nucleic acid into cells includes electroporation, calcium phosphate co-precipitation, mechanical techniques such as microinjection, transfer mediated by liposomes and direct DNA uptake and receptor-mediated DNA transfer.

As mentioned above, the aim of gene therapy using nucleic acid encoding a polypeptide, or an active portion thereof, is to increase the amount of the expression product of the nucleic acid in cells in which the level of the wild-type polypeptide is absent or present only at reduced levels. Such treatment may be therapeutic in the treatment of cells which are already cancerous or prophylactic in the treatment of individuals known through screening to have a susceptibility allele and hence a predisposition to, for example, cancer.

The present invention also provides a kit for generating a polynucleotide sequence or population of sequences of desired characteristics comprising reagents for ssDNA preparation, an exonuclease and components for carrying out a PCR technique, for example, thermostable DNA (nucleotides) and a stopping device, for example, EGTA.

As outlined above the present invention conveniently provides for the creation of mutated enzyme gene sequences and their random combination to functional enzymes having desirable characteristics. As an example of this aspect of the invention, the enzyme genes are mutated by error prone PCR which results in a mutation rate of approximately 0.7%. The resulting pool of mutated enzyme genes are then digested with an exonuclease, preferably BAL31, and the reaction inhibited by the addition of EGTA at different time points, resulting in a set of DNA fragments of different sizes. These may then be subjected to PCR based reassembly as described above. The resulting reassembled DNA fragments are then cloned and a gene library constructed. Clones may then be selected from this library and sequenced.

A further application of this technology is the generation of a population of variable DNA sequences which can be used for further selections and analyses. Besides encoding larger proteins, e.g. antibody fragments and enzymes, the DNA may encode peptides where the molecules functional characteristics can be used for the design of different selection systems. Selection of recombined DNA sequences encoding peptides has previously been described (Fisch *et al*., PNAS. USA 1996 Jul 23; 93 (15): 7761-7766). In addition, the variable DNA population can be used to produce a population of RNA molecules with e.g. catalytic activities. Vaish *et al*., (PNAS. USA 1998 Mar 3; 95 (5): 2158-2162) demonstrated the design of functional systems for the selection of catalytic RNA and Eckstein F (Ciba Found. Symp. 1997; 209; 207-212) has outlined the applications of catalytic RNA by the specific introduction of catalytic RNA in cells. The system may be used to further search through the sequence space in the selection of functional peptides/molecules with catalytic activities based on recombined DNA sequences.

Aspects and embodiments of the present invention will now be illustrated, by way of example, with reference to the accompanying figures. Further aspects and embodiments will be apparent to those skilled in the art.

### Brief description of the drawings

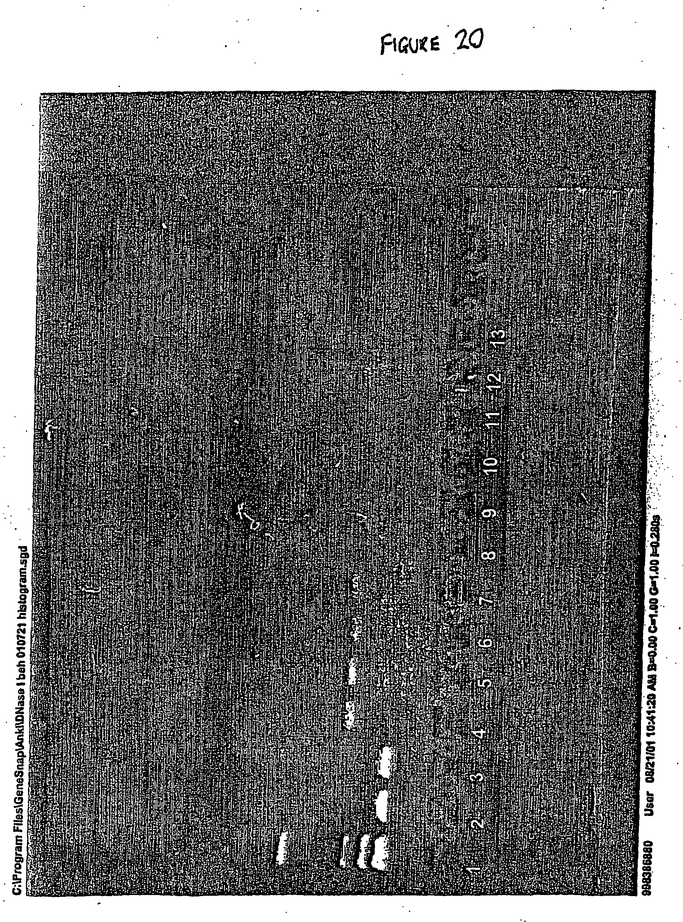

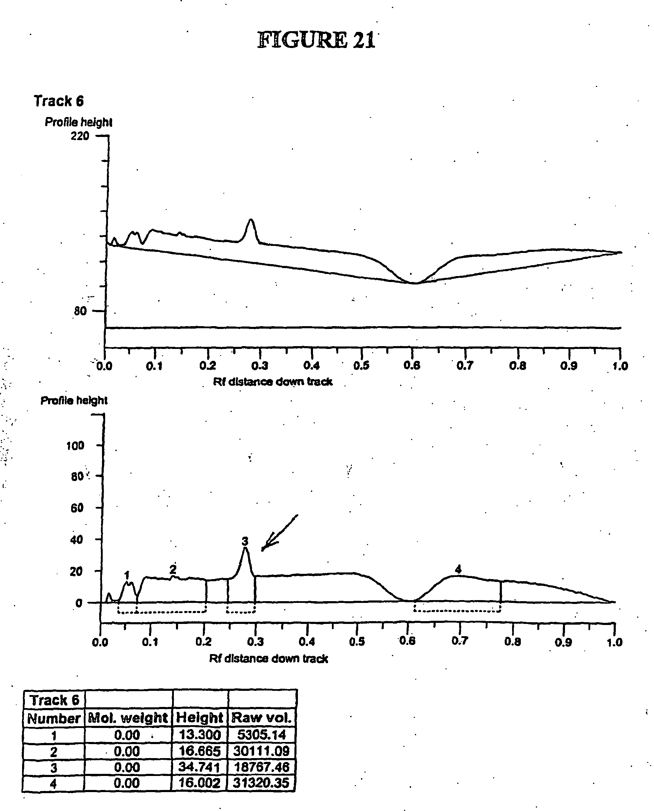

Figure 1 shows the principle of the method from template molecule to improved molecule.
Figure 2 shows the principle steps in preparation of single stranded DNA using biotin.
Figure 3 shows the principle steps in the preparation of single stranded DNA using phage.
Figure 4 shows the principle steps generating single stranded DNA fragments using exonuclease treatment.
Figure 5 shows the principle steps for assembly of single stranded DNA fragments using PCR.
Figure 6 shows the % of recombinants formed having one cross-over following digestion of dsDNA with 20 U/ml BAL31 for varying periods of time.
Figure 7 shows the % of recombinants formed having two cross-overs following digestion of dsDNA with 20 U/ml BAL31 for varying periods of time.
Figure 8 shows the % of recombinants formed having one cross-over following digestion of ssDNA with 1.25 U/ml BAL31 for varying periods of time.
Figure 9 shows the % of recombinants formed having two cross-overs following digestion of ssDNA with 1.25 U/ml BAL31 for varying periods of time.
Figure 10 shows the % of recombinants formed having one cross-over following digestion of ssDNA with 11 U/ml BAL31 for varying periods of time.
Figure 11 shows the % of recombinants formed having two cross-overs following digestion of ssDNA with 11 U/ml BAL31 for varying periods of time.
Figure 12 shows an agarose electrophoresis gel image of fragments generated following digestion of 300 ng ssDNA with BAL31 for 50 minutes (lane 1), 30 minutes (lane 2) and 10 minutes (lane 3). Untreated ssDNA is shown in lane 4. Molecular weight markers are shown in lane 5.
Figure 13 shows the corresponding gel chromatograms for lane 4 in Figure 12.
Figure 14 shows the corresponding gel chromatograms for lane 3 in Figure 12.
Figure 15 shows the corresponding gel chromatograms for lane 2 in Figure 12.
Figure 16 shows an agarose electrophoresis gel image of fragments generated following digestion of 300 ng DNA with exonuclease VII for 10 minutes (lane 3), 20 minutes (lane 4) and 30 minutes (lane 5). Untreated ssDNA is shown in lane 2. Molecular weight markers are shown in lane 1.
Figure 17 shows an agarose electrophoresis gel image of fragments generated following digestion of 300 ng DNA with exonuclease Rec J_{f} (9 U/mg ss/DNA) for 10 minutes (lane 2), 20 minutes (lane 3) and 30 minutes (lane 4). Untreated ssDNA is shown in lane 1. Molecular weight markers are shown in lane 5.
Figure 18 shows an agarose electrophoresis gel image of fragments generated following digestion of 300 ng DNA with exonuclease Rec J_{f} (36 U/mg ss/DNA) for 10 minutes (lane 3), 20 minutes (lane 4) and 30 minutes (lane 5). Untreated ssDNA is shown in lane 2. Molecular weight markers are shown in lanes 1 and 6.
Figure 19 shows an agarose electrophoresis gel image of fragments generated following digestion of 300 ng DNA with DNase I (0.15 U enzyme/mg DNA). Lane samples were as follows:
   - Lane 1:: Molecular weight markers
   - Lane 2:: Untreated ssDNA in Mg buffer
   - Lane 3:: ssDNA fragmented with DNase I in Mg buffer
   - Lane 4:: Untreated ssDNA in Mn buffer
   - Lane 5:: ssDNA fragmented with DNase I in Mn buffer
   - Lane 6:: (empty)
   - Lane 7:: (empty)
Figure 20 shows an agarose electrophoresis gel image of fragments generated following digestion of 300 ng DNA with DNase I. Lane samples were as follows:
   - Lane 1:: Molecular weight markers
   - Lane 2:: Untreated dsDNA in Mg buffer
   - Lane 3:: Untreated dsDNA in Mg buffer
   - Lane 4:: Untreated ssDNA (forward strand) in Mg buffer
   - Lane 5:: Untreated ssDNA (forward strand) in Mg buffer
   - Lane 6:: Untreated ssDNA (reverse strand) in Mg buffer
   - Lane 7:: Untreated ssDNA (reverse strand) in Mg buffer
   - Lane 8:: dsDNA fragmented with DNase I (0.24 U enzyme/µg DNA) in Mg buffer
   - Lane 9:: dsDNA fragmented with DNase I (1.3 U enzyme/µg DNA) in Mg buffer
   - Lane 10:: ssDNA (forward strand) fragmented with DNase I (0.24 U enzyme/µg DNA) in Mg buffer
   - Lane 11:: ssDNA (forward strand) fragmented with DNase I (1.3 U enzyme/µg DNA) in Mg buffer
   - Lane 12:: ssDNA (reverse strand) fragmented with DNase I (0.24 U enzyme/µg DNA) in Mg buffer
   - Lane 13:: ssDNA (reverse strand) fragmented with DNase I (1.3 U enzyme/µg DNA) in Mg buffer
Figure 21 shows the corresponding gel chromatograms for lane 6 in Figure 20.
Figure 21 shows the corresponding gel chromatograms for lane 6 in Figure 20.

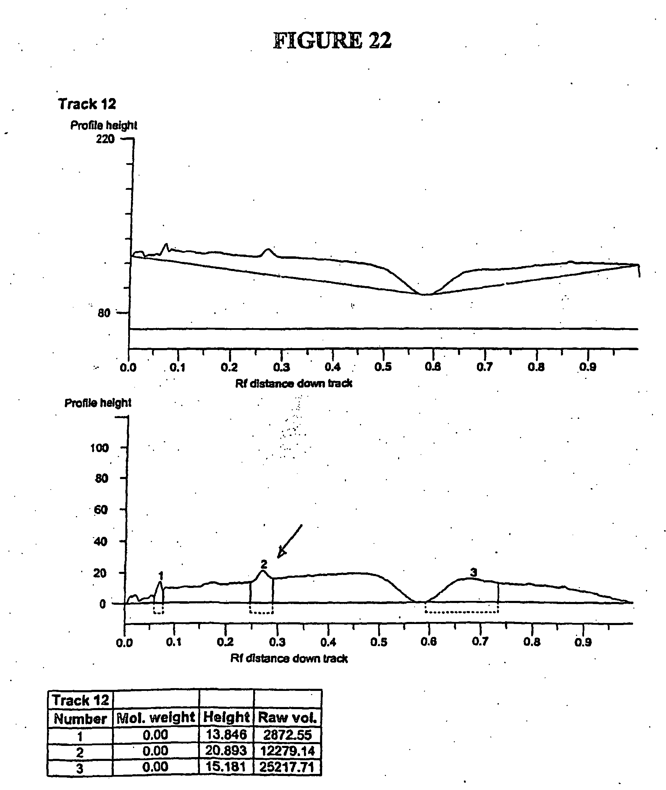

Figure 22 shows the corresponding gel chromatograms for lane 12 in Figure 20.

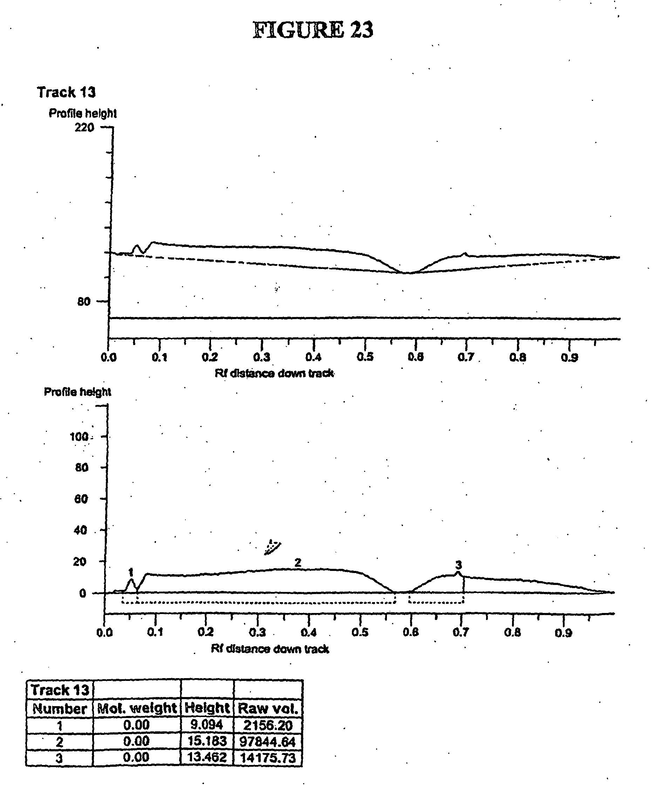

Figure 23 shows the corresponding gel chromatograms for lane 13 in Figure 20.

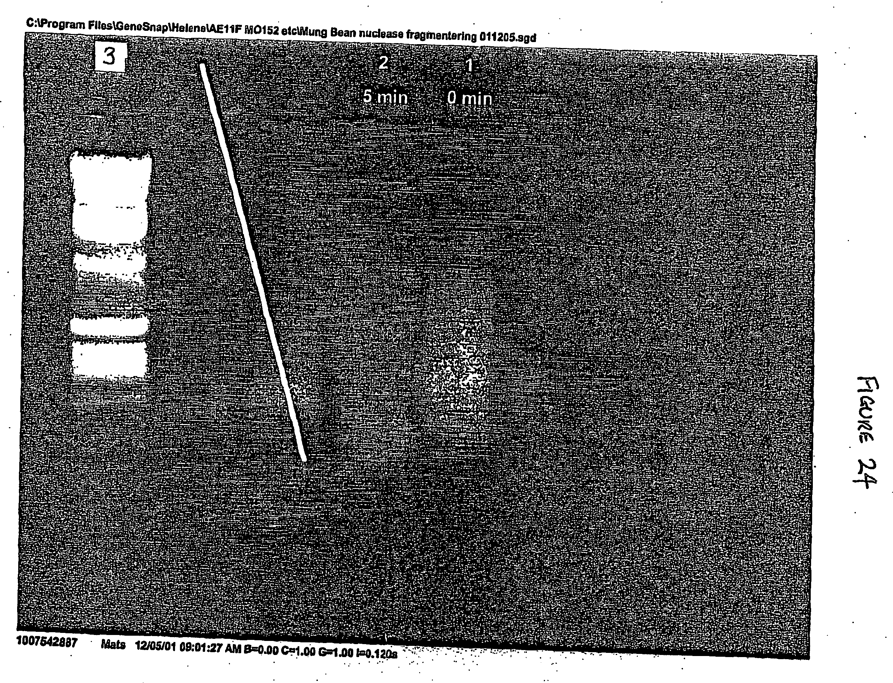

Figure 24 shows an agarose electrophoresis gel image of fragments generated following digestion of 300 ng DNA with Mung bean nuclease. Lane samples were as follows:
   - Lane 1:: Untreated ssDNA in Mg buffer
   - Lane 2:: ssDNA fragmented with Mung bean nuclease for 10 minutes
   - Lane 3:: Molecular weight markers

### EXAMPLES

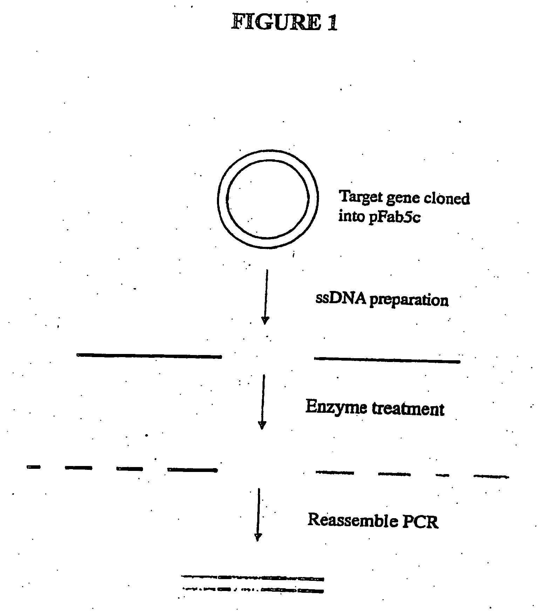

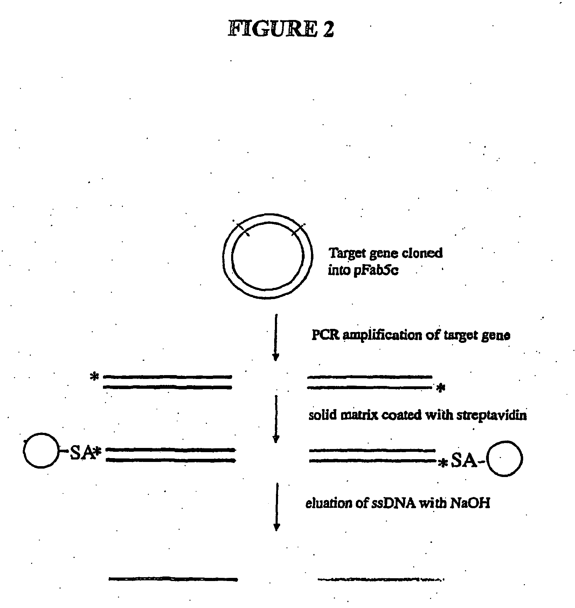

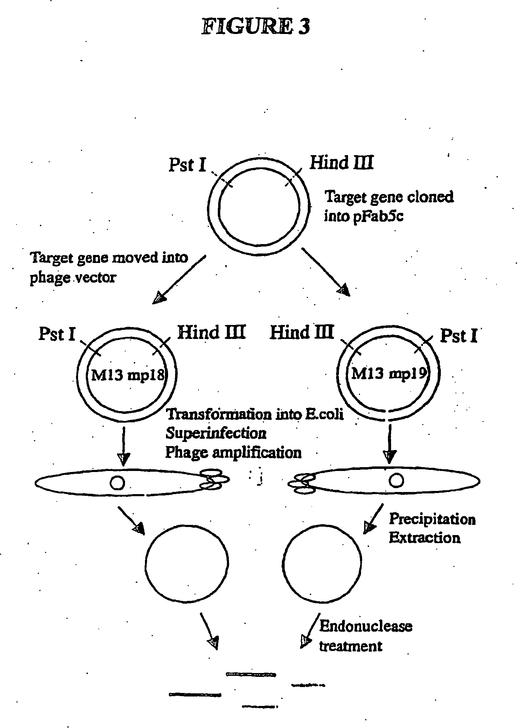

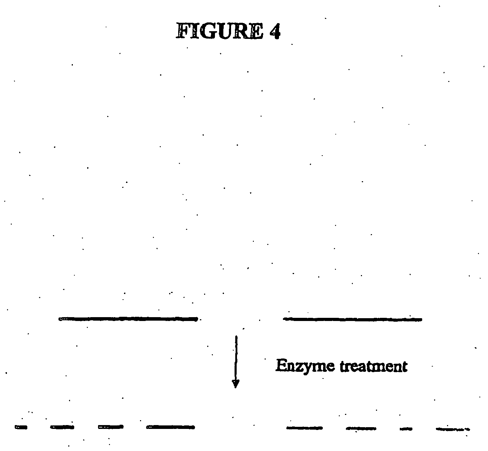

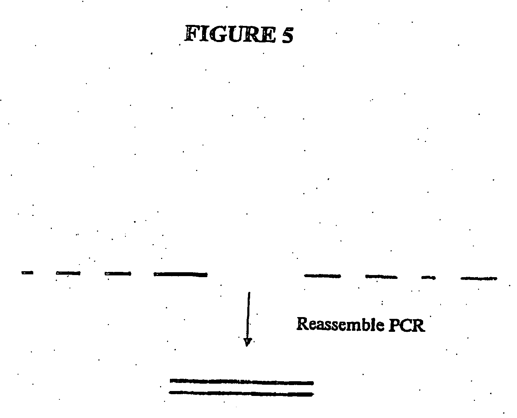

The DNA shuffling procedure can be illustrated by the steps shown in Figures 1 to 5. The gene encoding the protein of interest (X) in the plasmid pFab5chis is used in this example. Random mutations are introduced by error prone PCR. Single-stranded DNA is prepared. This can be carried out by either biotinylated primers or by the use of phage being able to pack single-stranded DNA, as discussed above. The coding and the non-coding ssDNA strands are prepared in different reactions (A and B). The ssDNA strands from either reactions are subjected to separate enzymatic treatment using e.g. BAL 31. By mixing the two pools of single-stranded DNA fragments in equimolar amounts the gene can be reassembled in a shuffled nature and in many versions by the use of two subsequent PCR reactions, where the first reaction contains no primers. After cloning this library of reassembled genes in pY, selections can be performed to achieve the improved molecule of interest.

A more detailed description of examples of the present invention is given below.

### Example 1

### Reagents

AmpliTaq® polymerase was purchased from Perkin-Elmer Corp., dNTPs from Boehringer Mannheim Biochemica (Mannheim, Germany), and BAL31 Nuclease from New England Biolabs Inc. (Beverly, USA). All restriction enzymes were purchased from New England Biolabs Inc. (Beverly, USA). Ethidium bromide was purchased from Bio-Rad Laboratories (Bio-Rad Laboratories, Hercules, CA, USA). T4 DNA Ligase was purchased from New England Biolabs Inc. (Beverly, USA). EDTA and EGTA was purchased from Kebo Lab (Sweden).

All primers were designed in the laboratory and obtained from Life Technologies (Täby, Sweden) and SGS-DNA (Köping, Sweden).

### PCR

All Polymerase Chain Reactions (PCR) were carried out in a automatic thermocycler (Perkin-Elmer Cetus 480, Norwalk, CT,USA). PCR techniques for the amplification of nucleic acid are described in US Patent No. 4,683,195. References for the general use of PCR techniques include Mullis *et al*., Cold Spring Harbor Symp. Quant. Biol., 51:263, (1987), Ehrlich (ed), PCR technology, Stockton Press, NY, 1989, Ehrlich *et al*., Science, 252:1643-1650, (1991), "PCR protocols; A Guide to Methods and Applications", Eds. Innis *et al*., Academic Press, New York, (1990).

### Sequencing

All constructs have been sequenced by the use of BigDye Terminator Cycle Sequencing kit (Perkin-Elmer, Elmervill, CA, USA). The sequencing was performed on a ABI Prism 377 DNA Sequencer.

### Agarose electrophoresis

Agarose electrophoresis of DNA was performed with 2% agarose gels (AGAROSE (FMC Bioproducts, Rockland, ME, USA)) with 0.25µg/ml ethidium bromide in Tris-acetate buffer (TAE-buffer 0.04M Tris-acetate, 0.001M EDTA). Samples for electrophoresis were mixed with a sterile filtrated loading buffer composed of 25% Ficoll and Bromphenolic blue and loaded into wells in a the 2% agarose gel. The electrophoresis was run at 90 V for 45 minutes unless otherwise stated in Tris-acetate buffer with 0.25 µg/ml ethidium bromide. Bands of appropriate size were gel-purified using the Qiaquick Gel Extraction Kit (Qiagen GmbH, Hilden, Germany) when needed. As molecular weight standard, DNA molecular weight marker 1 kb ladder (Gibco BRL) was used. The DNA-concentration of the gel extracted products were estimated using a spectrophotometer.

### Bacterial Strains

The *Escherichia* coli-strain TOP10F' was used as a bacterial host for transformations. Chemically competent cells of this strain were produced basically as described Hanahan, D. 1983. Studies on transformation of Escherichia coli with plasmids. J. Mol. Biol. 166: 557-580. Electrocompetent cells of this bacterial strain were produced (Dower, W.J., J. F. Miller, and C.W. Ragsdale. 1988: High efficiency transformation of *E.coli* by high voltage electroporation. Nucleic Acids Res. 16:6127).

### Plasmids

All genetic manipulations were performed in pFab5chis according to Molecular cloning; a laboratory manual (Second Edition, Cold Spring Harbor Laboratory Press, 1989). This vector is designed to harbour any scFv gene inserted between SfiI and NotI sites. The SfiI site is located in the pelB leader and the NotI site is located just after the VL region, such that VH-linker-VL is inserted. In this case, an antibody directed to CD40 was used.

### Primers

Two biotinylated primers surrounding the antibody gene of pFab5chis were designed with the following sequences including designated unique restriction sites:
1736 SfiI forward primer: 
5'-ATT ACT CGC GGC CCA GCC GGC CAT GGC CCA CAG GTC
AAG CTC GA
and 1735 NotI reversed primer:
   5'-TTA GAG CCT GCG GCC GCC TTG TCA TCG TCG TCC TT

Two non-biotinylated primers surrounding the antibody gene of pFab5chis were designed with the following sequences including designated unique restriction sites:
1664 SfiI forward primer: 
5'-ATT ACT CGC GGC CCA GCC GGC CAT GGC CCA CAG GTC
AAG CTC GA
and 1635 NotI reversed primer:
   5'-TTA GAG CCT GCG GCC GCC TTG TCA TCG TCG TCC TT

### Standard PCR

Standard PCR reactions were run at 25 cycles consisting of following profile: denaturation (94°C, 1 minute), primer annealing (55°C, 1 minute) and extension (72°C, 3 minutes). Each PCR reaction contained 10 mM Tris-HCl, pH 8.3, 50 mM KCl, 1.5 mM MgCl₂, 200 µM dNTP, 1 µM forward primer, 1 µM reverse primer, 1.25 U AmpliTaq® thermostable DNA polymerase (Perkin-Elmer Corp.), and 50 ng template in a final volume of 100 µl.

### Error Prone PCR

The error prone PCR reactions were carried out in a 10 x buffer containing 500 mM NaCl, 100 mM Tris-HCl, pH 8.8, 5mM MgCl₂ 100 µg gelatine (according to Kuipers *et al*., Nucleic Acids Res. 1991, Aug 25;19 (16):4558 but with MgCl₂ concentration increased from 2 mM to 5 mM).

For each 100 µl reaction the following was mixed:

| | |
|---|---|
| dATP 5 mM | 5 µl |
| dGTP 5 mM | 5 µl |
| dTTP 10 mM | 10 µl |
| dCTP 10 mM | 10 µl |
| 20 µM 3' primer | 1.5µl |
| 20 µM 5'-primer | 1.5 µl |
| 10x Kulpers buffer | 10 µl |
| sterile mp H₂O | 46.3 µl |

The template in pFab5chis vector was added at an amount of 50 ng. 10 µl of 10 mM MnCl₂ was added and the tube was checked that no precipitation of MnO₂ occurred. At last 5 Units of Taq enzyme was added. The error prone PCR was run at the following temperatures for 25 cycles without a hot start: 94°C 1', 45 °C 1', 72 °C 1' , + 72 °C for 7 minutes. The resulting product was an error proned insert over the protein of approximately 750 bp. This insert was purified with Gibco PCR purification kit, before further treatment.

### Generation of single stranded DNA by biotinylated primers

The fragment of interest was amplified by two separate PCR reactions. These reactions can be standard PCR as described above or error prone PCR also as described above. The primers should be designed so that in one reaction the forward primer is biotinylated and in the other reaction the reverse primer is biotinylated. For example, PCR reactions with A) primers 1736 and 1635 and B) primers 1664 and 1735, with the above mentioned profile was performed for 25 cycles with pFab5chis-antibody as template. This yielded PCR-products of approximately 750 bp where in A the upper strand was biotinylated and in B the lower strand was biotinylated.

The non-biotinylated strands were retrieved by purification using a solid matrix coated with streptavidin e.g. Dynabeads. The magnetic beads are washed and equilibrated with PBS/1% BSA and B&W buffer containing 5 mM Tris pH 7.5, 1 M NaCl, and 0.5 mM EGTA. 100 µl of each PCR product is mixed with 100 µl beads dissolved in 2 x B&W buffer and incubated at room temperature for 15 minutes with rotation. Unbound PCR products are removed by careful washing twice with B&W. The non-biotinylated strand of the captured DNA is eluted by alkaline denaturation by letting the DNA incubate with 25 µl 0.1 M NaOH for 10 minutes in room temperature. The solution is separated from the beads and neutralised with 7.5 µl 0.33 M HCl and 2.5 µl 1 M Tris pH 8.

### Generation of single stranded DNA using phage

The fragment of interest was cloned into bacteriophage M13 vectors M13mp18 and M13mp19 using PstI/HindIII restriction enzymes. The bacteriophage were propagated using *Escherichia coli-strain* TOP10F' according to conventional methods. Single stranded DNA for the upper strand was prepared from bacteriophage vector M13mp18 and single stranded DNA for the lower strand was prepared from bacteriophage vector M13mp19. Briefly, 1.5 ml of an infected bacterial culture was centrifuged at 12 000g for 5 minutes at 4°C. The supernatant was precipitated with 200 µl 20% PEG8000/2.5 M NaCl. The pelleted bacteriophage was resuspended in 100 µl TE. 50 µl phenol equilibrated with Tris-Cl (pH 8.0) was added and the sample was vortexed. After centrifugation at 12 000g for 1 minute at RT the upper phase, containing the DNA, was transferred and precipitated with ethanol. The DNA pellet was dissolved in 50 µl TE (pH 8.0) and stored at - 20°C. (Sambrook *et al*. Molecular Cloning, A laboratory manual 2^{nd} edition. Cold Spring Habor Laboratory Press. 1989, chapter 4). Single stranded DNA prepared from phage is circular and must be opened prior to BAL31 treatment. This can be performed with an endonuclease able to cleave single stranded DNA.

### Generation of single stranded fragmented DNA using BAL 31

The ssDNA strands from either reactions (containing upper and lower strands, respectively) were subjected to separate enzymatic treatment using e.g. BAL 31. Each digestion reaction contained 0.02 µg/µl ssDNA, 600 mM NaCl, 20 mM Tris-HCl, 12 mM CaCl₂, 12 mM MgCl₂, 1 mM EDTA pH 8.0 and BAL 31 at various enzyme concentrations ranging from 0.1 - 5 U/ml. The reactions were incubated at 30°C and fractions of digested ssDNA were collected sequentially at 10, 30, 60 and 120 seconds or longer. The reactions were stopped by addition of EDTA and heat treatment at 65°C for 10 minutes. The ssDNA fragments were purified by phenol/chloroform extraction and ethanol precipitated. The ssDNA are resuspended in 10 mM Tris pH 8.0.

The digestion pattern was evaluated by 1 % agarose gel electrophoresis.

### Purification of digestion produced fragments:

Digested DNA fragments were purified by phenol/chloroform/isoamylalcohol extraction. 50 µl of buffered phenol was added to each tube of 100 µl sample together with 50 µl of a mixture of chloroform and isoamylalcohol (24:1). The tubes were vortexed for 30 seconds and then centrifuged for 1 minute in a microfuge at 14000 r.p.m. The upper phase was then collected and mixed with 2.5 volumes of 99.5% Ethanol (1/10 was 3M Sodium Acetate, pH 5.2). The DNA was precipitated for 1 hour in -80 °C. The DNA was then pelleted by centrifugation for 30 minutes in a microfuge at 14.000 r.p.m. The pellet was washed once with 70% ethanol and then re-dissolved in 10 µl of sterile water.

### Analysis of digestion produced purified fragments on agarose gel

5 µl of the dissolved pellet from each time point and from the blank were mixed with 2.5 µl of loading buffer (25% Ficoll and Bromphenolic blue) and loaded into wells in a 2% agarose gel. The electrophoresis of the different time points were performed as above.

### Reassembly of full length fragments

Reassembly of the ssDNA fragments is achieved by two sequential PCR reactions. The first PCR reaction should contain 10 mM Tris-HCl, pH 8.3, 50 mM KCl, 1.5 mM MgCl₂, 200 µM dNTP, 0.3 U Taq polymerase and 2 µl BAL31 treated sample, all in a final volume of 25 µl, and subjected to 5 cycles with the following profile: 94 °C for 1 minute, 50 °C for 1 minute and 72 °C for 2 minutes + 72 °C for 5 minutes. The second PCR reaction should contain 10 mM Tris-HCl, pH 8.3, 50 mM KCl, 1.5 mM MgCl₂, 200 µM dNTP, 0.6 U Taq polymerase, 1 µM forward primer, 1 µM reverse primer, and 5 µl sample from the first PCR reaction, all in a final volume of 50 µl, and subjected to 15 cycles with the following profile: 94 °C for 1 minute, 55 °C for 1 minute and 72 °C for 2 minutes + 72 °C for 7 minutes. The resulting products can be evaluated by agarose gel electrophoresis.

### Restriction digestion of reassembled fragment and plasmid with SfiI and NotI

The reassembled fragment and the plasmid pFab5chis were first cleaved with SfiI by using NEB buffer 2 including BSA and 11 U enzyme/µg DNA. The reaction was carried out for 4 h at 50°C. After this the DNA was cleaved with NotI by adding conversion buffer and 6 U enzyme/µg DNA. This reaction was carried out for 37°C overnight.

### Gel purification of restriction digested vector and restriction digested reassembled fragment

The cleavage reactions were analysed on a 1% agarose gel. The restriction digested insert showed a cleavage product of about 750 bp. This corresponds well with the expected size. The band of the cleaved insert and plasmid was cut out and gel-extracted as previously described.

### Ligation of reassembled restriction digested fragment with restriction digested pFab5chis

Purified cleaved pFab5chis was ligated with purified reassembled restriction digested fragment at 12°C water bath for 16 hours. 50 µl of the vector was mixed with 50 µl of the insert and 15 µl of 10x buffer (supplied with the enzyme), 7.5µl ligase (5 U/µl) and sterile water to a final volume of 150µl. A ligation of restriction digested pFab5chis without any insert was also performed in the same manner.

### Transformation of chemically competent E coli TOP10F' with the ligated reassembled insert and pFab5chis

The ligation reactions were purified by phenol/chloroform extraction as described above. The upper phase from the extraction was collected and mixed with 2.5 volumes of 99.5% Ethanol (1/10 was 3M Sodium Acetate, pH 5.2). The DNA was precipitated for 1 hour in -80 °C. The DNA was then pelleted by centrifugation for 30 minutes in a microfuge at 14.000 r.p.m. The pellet was washed once with 70% ethanol and then re-dissolved in 10 µl of sterile water. 5 µl of each ligation was separately mixed with 95 µl chemically competent *E coli* TOP10F' incubated on ice for 1 hour and then transformed (Sambrook *et al*. Molecular Cloning, A laboratory manual 2^{nd} edition. Cold Spring Habor Laboratory Press, 1989). After one hour's growth the bacteria from the two transformations were spread onto ampicillin containing agar plates (100 µg/ml). The plates were grown upside-down in a 37°C incubator for 14 hours.

### Example 2 - Recombination frequencies; comparison of dsDNA and ssDNA

In further comparable experiments, three scFv antibody fragments were used in a recombination experiments, either as dsDNA or as ssDNA.

### dsDNA

The three scFv genes were each amplified in PCR using forward and reverse primers and standard PCR procedure. The size of the bands was confirmed with agarose electrophoresis and the rest of the amplified PCR products were purified using Concert PCR purification kit (Gibco). The dsDNA from the three scFv were mixed in equimolar amounts and treated with BAL31. Each digestion reaction contained dsDNA at a concentration of 0.02 µg/µl reaction volume, 600 mM NaCl, 20 mM Tris-HCl, 12 mM CaCl₂, 12 mM MgCl₂, 1 mM EDTA pH 8.0 and BAL31 at various enzyme concentrations (using 4, 20 or 100 U enzyme/ml reaction volume). The reactions were incubated at 30°C and fractions of digested dsDNA were collected sequentially at 10, 30, and 50 minutes. The reactions were stopped with EDTA and heat treatment (alternatively, an EDTA-free heat inactivation protocol may be used; see below) and purified using phenol/chloroform extraction and ethanol precipitation. The dsDNA samples were resuspended in 10 mM Tris pH 8.0.

Keeping each time point separate, the samples were subjected to reassembly PCR (for this reassembly 60 ng DNA is used) and amplification PCR according to the protocol, and cloned in pGEM (Product No A362A, Promega, Madison, USA). Eighteen clones from each time point were sequenced and the number and frequency of recombinations were determined.

### Heat inactivation of exonuclease digestions

A protocol to stop the BAL31 reaction without using EDTA has been established. This heat inactivation protocol avoids using phenol/chloroform extraction, which is hazardous to health and also causes loss of material.

In brief, the sample is incubated for 10 minutes at 95 C and then put directly in ice, to stop the enzymatic reaction. After this the sample can be directly precipitated using ethanol.

### ssDNA

The three scFv genes were each amplified in two PCR reactions using primer pairs forward/reverse-biotin and forward-biotin/reverse using standard PCR procedure. The size of the bands were confirmed with agarose electrophoresis and the rest of the amplified PCR products were purified using Concert PCR purification kit (Gibco). Single stranded DNA was obtained using magnetic beads according to the protocol, achieving three sense strands and three antisense strands. The sense strands and the antisense strands, respectively, from the three scFv were mixed in equimolar amounts and treated with BAL31 according to the protocol (using 1.25 or 11 U enzyme/ml reaction volume and ssDNA at a concentration of 0.015 µg/µl reaction volume) and samples were taken out at 0 (*i*.*e*. undigested), 10, 30 and 50 minutes. The reactions were stopped with EDTA and heat treatment and purified using phenol/chloroform extraction and ethanol precipitation. Keeping each time point separate, but mixing sense and antisense strands, the samples were subjected to reassembly PCR (for this reassembly 60 ng DNA is used) and amplification PCR according to the protocol, and cloned in pGEM. Eighteen clones from each time point were sequenced and the number and frequency of recombinations were determined.

### Results

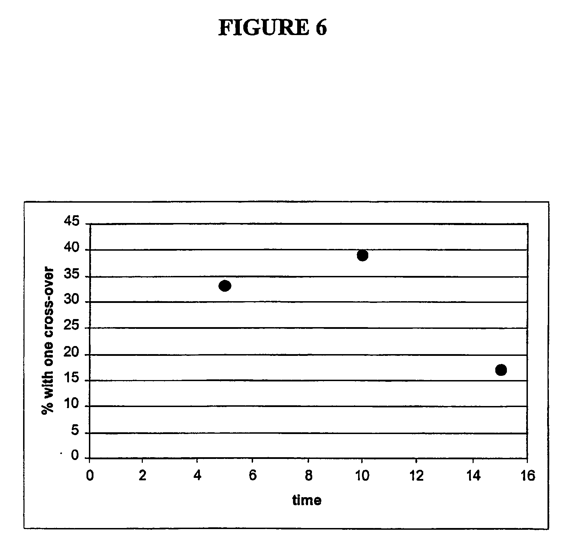

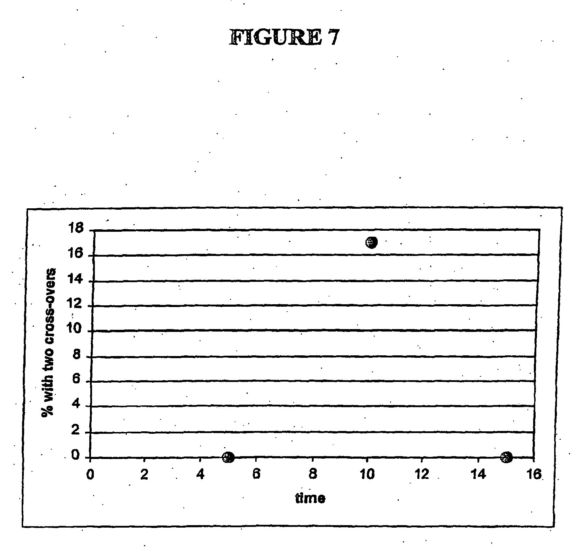

The highest frequency of recombination using dsDNA was achieved using 20 U enzyme/ml reaction volume (containing 0.02 µg/µl DNA) and treating for 10 minutes. This gave 39% of the clones with one cross-over (Figure 6) and 17% of the clones with two cross-overs (Figure 7). Using 4 U enzyme/ml gave no cross-overs independent of time for fragmentation and 100 U enzyme/ml resulted in complete fragmentation into very small fragments, as indicated by the failure to regain the full-length gene during reassembly.

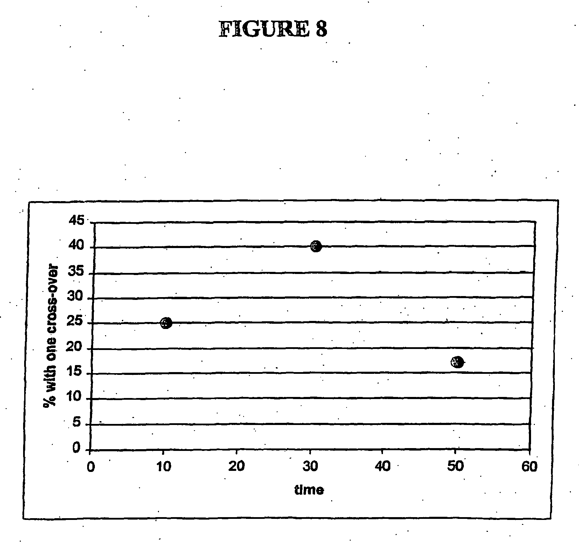

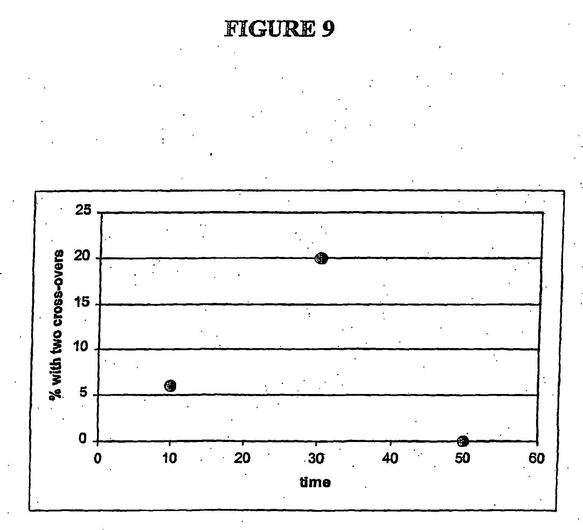

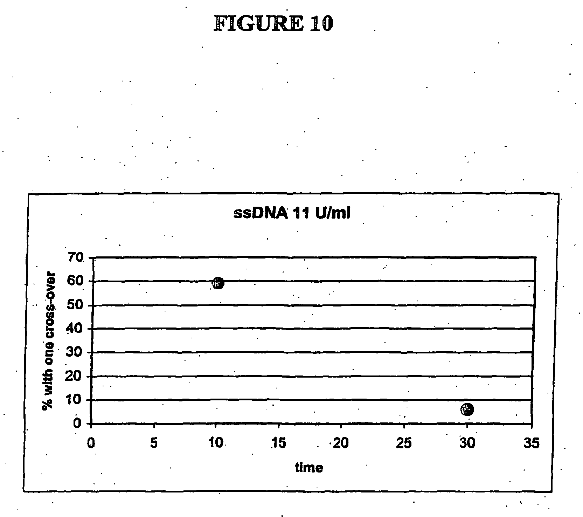

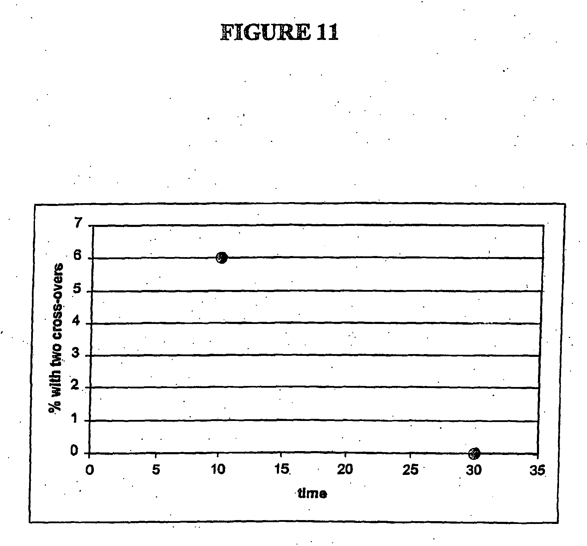

The results from the experiments using ssDNA are shown in Figures 8 to 10. Figure 8 shows 1.25 U/ml BAL31 and clones with one cross-over, Figure 9 shows 1.25 U/ml BAL31 and clones with two cross-overs. Figure 10 shows 11 U/ml BAL31 and clones with one cross-over, and Figure 11 shows 11 U/ml BAL31and clones with two cross-overs.

The highest frequency of recombination giving one cross-over using ssDNA was achieved using 11 U enzyme/ml and treating for 10 minutes (Figure 10). 59% of the clones had one cross over. The highest frequency of recombination giving two cross-overs using ssDNA was achieved using 1.25 U enzyme/ml and treating for 30 minutes (Figure 9). 20% of the clones had two cross overs.

### Conclusions and comments

These data clearly show that a higher frequency of recombination is achieved using ssDNA. The three scFv used have the same framework sequences, indicating that the number of cross overs reported may be higher due to cross overs in regions where no sequence difference will result. These experiments using ssDNA were carried out in a non-optimal fashion for showing maximum recombination, since all strands from all three molecules were mixed. Mixing the sense strand from one scFv with the antisense strand from another scFv would produce higher frequencies of cross overs, see Example 3 below. Also, each time point was here kept separate and it would be logical to estimate the frequency of cross overs to increase if different time points, i.e. different fragments sizes, are mixed.

### Example 3 - Recombination frequencies; homology dependence using ssDNA

To investigate the homology required to achieve cross-over we set up experiments to recombine four scFv (designated SMUC159, CT17, AE11 and MO152) making up three pairs with different homologies, as follows:

| | |
|---|---|
| SMUC159 - CT17 | 92% |
| SMUC159 - AE11 | 70% |
| SMUC159 - MO152 | 60% |

The four scFv genes were each amplified in two PCR reactions using primer pairs forward/reverse-biotin and forward-biotin/reverse using standard PCR procedure. The size of the bands were confirmed with agarose electrophoresis and the rest of the amplified PCR products were purified using Concert PCR purification kit (Gibco). Single stranded DNA was obtained using magnetic beads according to the protocol, achieving four sense strands and four antisense strands. Each strand was treated with BAL31 according to the protocol (using 4.2 or 12.5 U enzyme/ml) and samples were taken out at 0, 10, 30 and 50 minutes, or 0, 15, 30, 45 and 60 minutes. The reactions were stopped with EDTA and heat treatment and purified using phenol/chloroform extraction and ethanol precipitation. Keeping each time point separate, but mixing sense and antisense strands forming the pairs as indicates above, the samples were subjected to reassembly PCR and amplification PCR according to the protocol, and cloned in pGEM. Fifteen clones from each time point were sequenced and the number and frequency of recombination were determined.

### Results

Cross overs were identified in all combinations of scFv, indicating that as low as 60% homology is enough to achieve recombination.

### Example 4 - Improved control of fragment size using exonucleases

### (A) Exonucleases

We use exonucleases, e.g. BAL31, exonuclease I, exonuclease V, exonuclease VII, and exonuclease Rec J_{f} for fragmentation in the methods of the present invention. These enzymes cleave off one nucleotide at a time, either from the 5' end or from the 3'end or from both ends. The reaction can be stopped using EDTA or heat inactivation (see above), depending on the enzyme used. This means that fragments of all possible sizes, differing with only one nucleotide, can be obtained.

The following examples demonstrate how exonuclease digestion allows the creation of fragments of various and controllable sizes depending on the conditions used.

### BAL 31

Single-stranded DNA was digested with BAL31 according to the protocol in Example 1, with an enzyme concentration of 4.2 U/ml reaction volume and ssDNA concentration of 0.008 µg/µl reaction volume.

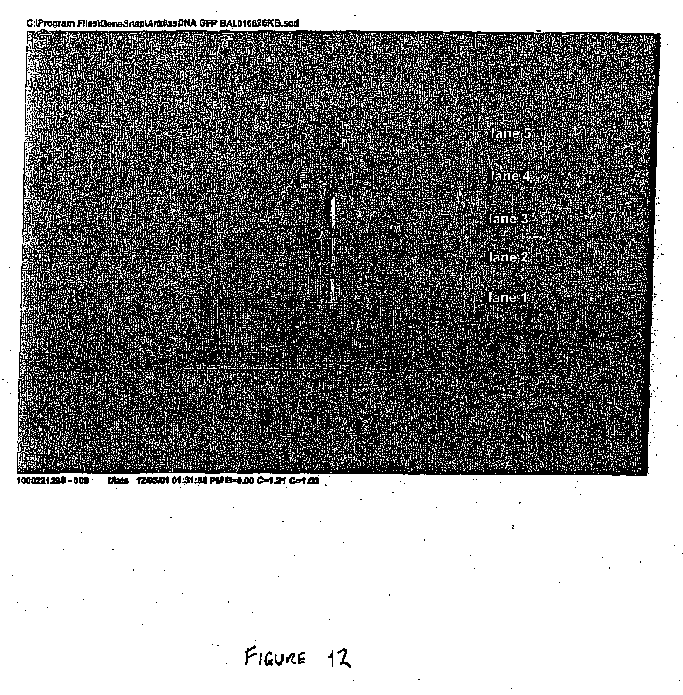

In a typical experiment, about 300 ng DNA is isolated at each time point of BAL31 treatment. Figure 12 shows an agarose electrophoresis gel image of such an experiment with untreated ssDNA in lane 4 and ssDNA treated for 10 minutes in lane 3, for 30 minutes in lane 2 and for 50 minutes in lane 1. Lane 5 is the molecular weight (MW) standard.

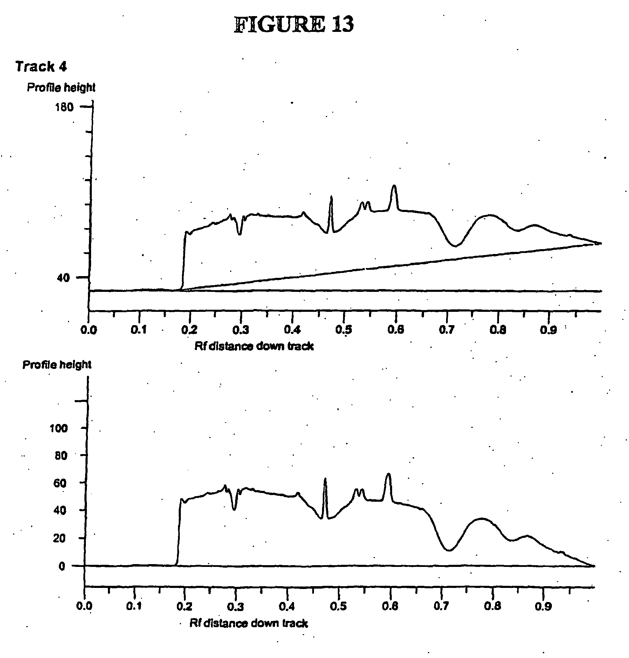

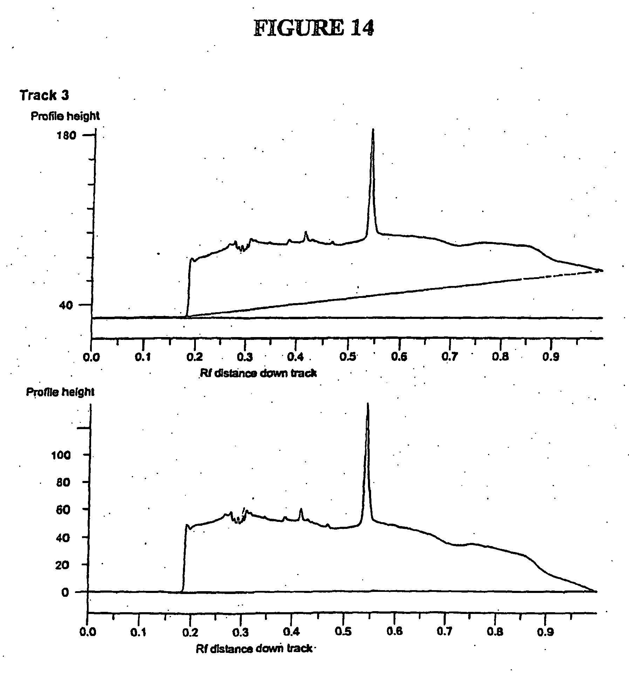

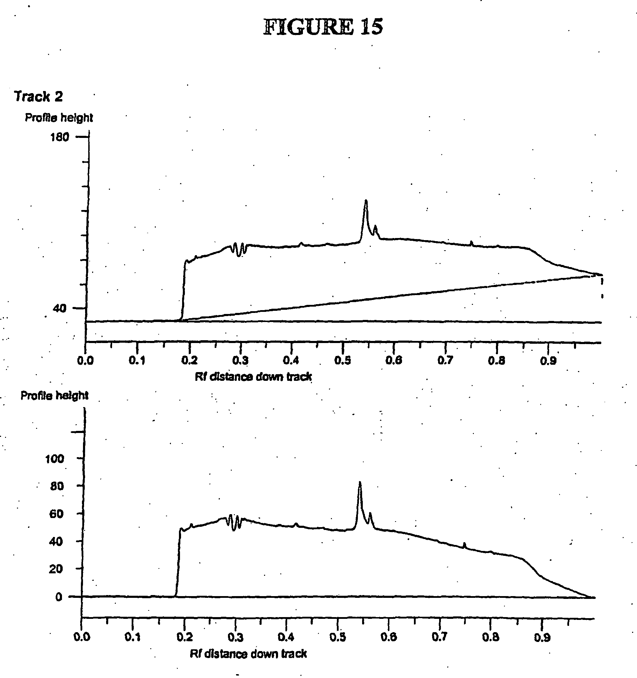

Figures 13 to 15 shows the corresponding gel chromatograms of the lanes, respectively. Figure 13 is the untreated material and the multiple peaks refer to different conformations of the ssDNA. Figure 14 corresponds to lane 3 and material treated for 10 minutes. The material was heat treated to stop the enzymatic reaction, and thus resolving the different conformations, and one peak of a distinct size is shown. Figure 15 corresponds to lane 2 and material treated for 30 minutes.

Here it is clear that the peak corresponding to larger fragments is decreasing and a peak of smaller DNA fragments has appeared.

### Exonuclease VII

Single-stranded DNA was digested with exonuclease VII using an enzyme concentration of 7.7 U/ml reaction volume and ssDNA concentration of 0.008 µg/µl reaction volume. The reaction buffer comprised 67 mM potassium phosphate (pH 7.9), 10 mM mercaptoethanol, 6.7 mM MgCl₂ and 8.3 mM EDTA.

The reaction was allowed to proceed at 37°C for 10, 20 and 30 minutes, before being stopped by heat inactivation (95°C for 5 minutes).

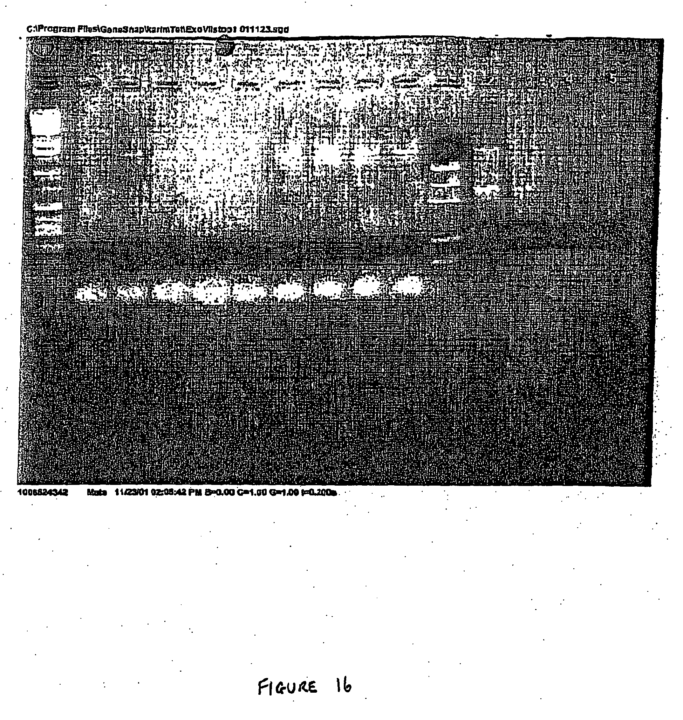

In Figure 16 the fragmentation pattern using exonuclease VII is shown. Lane 1 is MW standard, lane 2 is untreated ssDNA, lane 3 is ssDNA fragmented with exonuclease VII for 10 minutes, lane 4 is ssDNA fragmented with exonuclease VII for 20 minutes, and lane 5 is ssDNA fragmented with exonuclease VII for 30 minutes. This shows that the fragment sizes are decreased by time.

### Exonuclease Rec J_{f}

Single-stranded DNA was digested with exonuclease Rec J_{f} using an enzyme concentration of either 2.5 U/ml reaction volume or 10 U/ml reaction volume and ssDNA at a concentration of 0.007 µg/µl reaction volume, corresponding to 0.36 U enzyme/µg DNA and 1.4 U enzyme/µg DNA, respectively. The reaction buffer comprised 50 mM NaCl, 10 mM Tris.HCl, 10 mM MgCl₂ and 1 mM dithiothreitol, at pH 7.9

The reaction was allowed to proceed at 37°C for 10, 20 and 30 minutes, before being stopped by heat inactivation (95°C for 5 minutes).

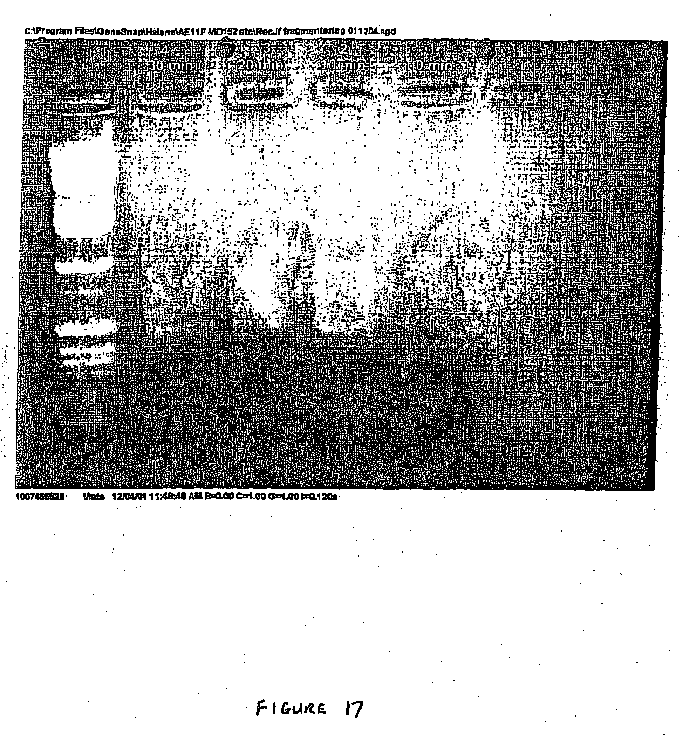

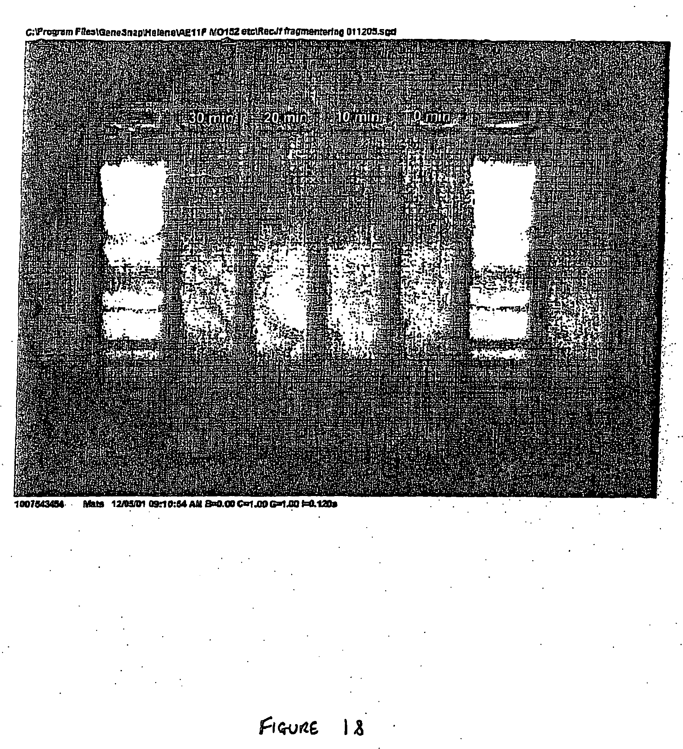

In Figure 17 the fragmentation pattern using exonuclease Rec J_{f} at 036 U/microgram ssDNA is shown. Lane 1 untreated ssDNA, lane 2 is ssDNA fragmented with exonuclease Rec J_{f} for 10 minutes, lane 3 is ssDNA fragmented with exonuclease Rec J_{f} for 20 minutes, and lane 4 is ssDNA fragmented with exonuclease Rec J_{f} for 30 minutes. This shows that the fragment sizes are decreased by time. In Figure 18 the enzyme concentration is increased 4 times (1.4 U/microgram ssDNA) and the fragmentation pattern is shown from 0 to 30 minutes, showing a higher degree of fragmentation as compared to Figure 17. This shows that both time and enzyme concentration can be used to control the fragmentation.

### (B) Endonucleases

Conventional DNA shuffling methods typically use DNase I for fragmentation (for example, see Stemmer, 1994, *Nature* **370**:389-391). DNase I cleaves DNA in an endonucleolytic fashion at sites adjacent to pyrimidines. Consequently, not all possible fragment sizes can be obtained.

Moreover, using magnesium in the reaction buffer, a homologous mix of mono- and oligomers is obtained. Hence, different methods such as gel agarose electrophoresis purification or gel filtration need to be used in order to isolate fragments of different sizes. Often fragments of small size or a mix of small and larger fragments are desired to optimise recombination. However, these purification methods introduce single-stranded nicks in the double-stranded PCR products. Fragments of a particular size purified on a gel would thus consist of dsDNA with a large number of single-stranded nicks, which would give rise to many smaller fragments upon denaturation. This means that many of the single-stranded fragments generated upon denaturation would be too short to function as primers during the annealing, resulting in a great loss of product.

Using manganese in the reaction buffer creates fragments of sizes smaller than 50 bp and no gel purification is needed. However, here you are restricted to use only small fragments and these can not be mixed with larger fragments, something that would probably increase the recombination frequency.

The problems associated with the use of endonucleases are demonstrated in the following experiments:

### DNase I

DNA was digested for 5 minutes with DNase I at a concentration of 0.15 U/µg DNA.

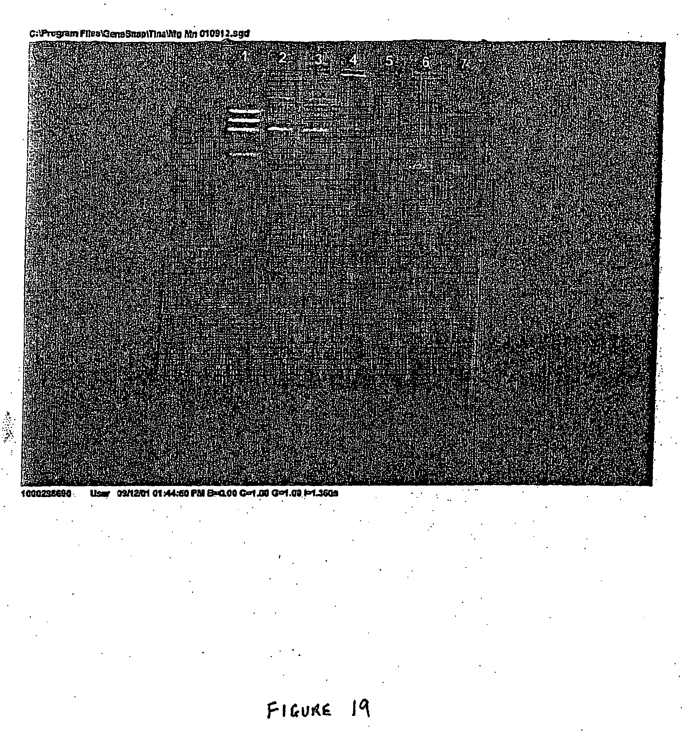

Magnesium and manganese buffers were compared when fragmenting with DNase I and the result is shown in Figure 19. Lane 1 is MW standard, lane 2 is untreated ssDNA in Mg buffer, lane 3 is ssDNA fragmented with DNase I in Mg buffer according to Stemmer (1994) *Nature* **370**:389-391, lane 4 is untreated ssDNA in Mn buffer and lane 5 is ssDNA fragmented with DNase I in Mn buffer according to *Kikuchi et al*. (2000) *Gene* **243**:133-137. It is clear from Figure 19 that, when using Mg buffer and conditions according to the Stemmer and Kikuchi papers, no fragmentation occurs. Moreover, when using Mn buffer and conditions according to the Stemmer and Kikuchi papers, all material is totally fragmented within only a few minutes.

In an attempt to obtain fragments of different sizes we decided to use Mg buffer and increase the enzyme concentration. Figure 20 shows an agarose electrophoresis gel image of such an experiment using DNase I. Lane 1 is the MW standard. Lane 6 is untreated ssDNA. Lane 12 is ssDNA treated according to the Stemmer and Kikuchi papers, using 0.15 U enzyme/microgram DNA and lane 13 is the same material treated with 1 U enzyme/microgram DNA (i.e. six times more enzyme).

Figures 21 to 23 shows the corresponding chromatograms. The untreated ssDNA has been heat treated, therefore only one peak appears in Figure 21 (indicated by arrow). In figure 22, it is apparent that using the amount of DNase I according to the Stemmer and Kikuchi papers the peak for untreated ssDNA is somewhat decreased (indicated by arrow) but no distinct peak is visible for the fragmented DNA, only a smear. Using 6 times more enzyme the untreated ssDNA is totally abolished (Figure 23) and neither here is any visible peak of the fragments.

### Mung bean nuclease

Single-stranded DNA was digested with Mung bean nuclease (Product No MO250S, New England Biolabs) using an enzyme concentration of either 0.375 U/ml reaction volume and ssDNA at a concentration of 0.007 µg/µl reaction volume. The reaction buffer comprised 50 mM sodium acetate, 30 mM NaCL, 1 mM ZnSO₄, at pH 5.0.

The reaction was allowed to proceed at 25°C for 10 minutes, before being stopped by heat inactivation (95°C for 5 minutes).

Figure 24 shows fragmentation using another endonuclease, Mung bean nuclease. Lane 1 is the untreated ssDNA, lane 2 is the same material treated for 10 minutes. Lane 3 is the MW standard.

Results indicate that all DNA was totally fragmented after only 10 minutes digestion with Mung bean nuclease (see lane 2), despite using the enzyme at a concentration lower than that recommended by the manufacturer.

### Conclusions and comments

The above examples show how the fragment sizes can be controlled using exonucleases and altering the reaction conditions, *i*.*e*. time, reaction volume, enzyme concentration. The different peaks are visualised using gel image chromatograms.

In contrast, using endonucleases, such as DNase I, gives a reaction which is hard to control. Using conditions as referred in the literature, either using Mg or Mn containing buffers, typically gives a situation when either everything or nothing is fragmented, see especially Figure 20. An experiment using another endonuclease (Mung bean nuclease) confirms these observations.

## Claims

1. A method for generating a polynucleotide sequence or population of sequences from parent single stranded polynucleotide sequences encoding one or more protein motifs, comprising the steps of
a) providing single stranded DNA constituting plus and minus strands of parent polynucleotide sequences;
b) digesting the single stranded polynucleotide sequences with an exonuclease to generate populations of single stranded fragments;
c) contacting said fragments generated from the plus strands with fragments generated from the minus strands and optionally, adding primer sequences that anneal to the 3'and 5'ends of at least one of the parent polynucleotides under annealing conditions;
d) amplifying the fragments that anneal to each other to generate at least one polynucleotide sequence encoding one or more protein motifs having altered characteristics as compared to the one or more protein motifs encoded by said parent polynucleotides.

2. A method as claimed in Claim 1 wherein the exonuclease is selected from the group consisting of BAL31, exonuclease I, exonuclease V, exonuclease VII, and exonuclease Rec J_{f}

3. A method as claimed in Claim 2 wherein the exonuclease is BAL31.

4. A method as claimed in Claim 1, 2 or 3 wherein a parent polynucleotide sequence or sequences has been subjected to mutagenesis.

5. A method as claimed in Claim 1 wherein the population of fragments generated in step b) are subjected to mutagenesis.

6. A method as claimed in Claim 4 or 5 wherein the mutagenesis is error prone PCR.

7. A method as claimed in any one of Claims 1 to 6 wherein step b) is carried out to generate a population of single-stranded fragments of varying lengths.

8. A method as claimed in Claim 7 wherein step b) is controlled to generate a population of single-stranded fragments having an average length of more than approximately 50 nucleotides.

9. A method as claimed in any one of Claims 1 to 8 further comprising the step of expressing at least one polynucleotide sequence generated in step d) to produce the encoded polypeptide.

10. A method as claimed in Claim 9 further comprising the step of testing the encoded polypeptide for desired characteristics.

11. A method as claimed in any one of Claims 1 to 10 wherein the parent polynucleotide sequence encodes an antibody or fragment thereof.

12. A method as claimed in any one of Claims 1 to 10 wherein the parent polynucleotide sequence encodes an enzyme.

13. A method as claimed in any one of Claims 1 to 10 wherein the parent polynucleotide sequence encodes an antigen.

14. A method as claimed in any one of Claims 1 to 13 further comprising the step of incorporating the at least one polynucleotide sequence generated in step d) into a vector.

15. A method as claimed in Claim 14 further comprising the step of expressing in vitro the at least one polynucleotide sequence incorporated in a vector to produce the encoded polypeptide.

16. A method as claimed in Claim 14 or 15 further comprising the step of formulating the polynucleotide sequence or vectors comprising the polynucleotide or the polypeptide encoded by the polynucleotide into a pharmaceutically acceptable composition.

## Patentansprüche

1. Verfahren zum Erzeugen einer Polynukleotidsequenz oder einer Population von Polynukleotidsequenzen aus einer Einzelstrangausgangspolynukleotidsequenz, die ein oder mehrere Proteinmotive codiert, mit den Schritten:
a) Bereitstellen von Einzelstrang-DNS, die Plus- und Minusstränge von Ausgangspolynukleotidsequenzen ausbildet;
b) Aufschließen der Einzelstrangpolynukleotidsequenzen mit einer Exonuklease, um Populationen von Einzelstrangfragmenten zu erzeugen;
c) Kontaktieren der Fragmente, die aus den Plussträngen erzeugt wurden, mit Fragmenten, die aus den Minussträngen erzeugt wurden, und optionales Hinzugeben von Primersequenzen, die sich and die 3'- und 5'-Enden mindestens eines der Ausgangspolynukleotide unter Anlassbedingungen anlassen;
d) Verstärkung von Segmenten, die sich aneinander anlassen, um mindestens eine Polynukleotidsequenz zu erzeugen, die ein oder mehrere Proteinmotiv(e) codiert, das/die verglichen mit dem einen oder den mehreren Proteinmotiv(en), welche(s) durch die Ausgangspolynukleotide codiert wird/werden, geänderte Eigenschaften aufweist/ausweisen.

2. Verfahren nach Anspruch 1, wobei die Exonuklease ausgewählt ist aus der Gruppe, die besteht aus BAL31, Exonuklease I, Exonuklease V, Exonuklease VII, und Exonuklease Rec J_{f.}.

3. Verfahren nach Anspruch 2, wobei die Exonuklease BAL 31 ist.

4. Verfahren nach Anspruch 1, 2 oder 3, wobei eine Ausgangspolynukleotidsequenz oder Ausgangspolynukleotidsequenzen einer Mutationserzeugung unterworfen worden ist/sind.

5. Verfahren nach Anspruch 1, wobei die Population von Fragmenten, die in Schritt b) erzeugt worden ist, einer Mutationserzeugung unterworfen wird.

6. Verfahren nach Anspruch 4 oder 5, wobei die Mutationserzeugung zu Fehlern neigende PKR ist.

7. Verfahren nach einem der Ansprüche 1 bis 6, wobei Schritt b) durchgeführt wird, um eine Population von Einzelstrangfragmenten von variierender Länge zu erzeugen.

8. Verfahren nach Anspruch 7, wobei Schritt b) gesteuert wird, um eine Population von Einzelstrangfragmenten mit einer mittleren Länge von mehr als ungefähr 50 Nukleotiden zu erzeugen.

9. Verfahren nach einem der Ansprüche 1 bis 8, das weiterhin den Schritt des Exprimierens mindestens einer Polynukleotidsequenz aufweist, die in dem Schritt d) erzeugt wurde, um das codierte Polypeptid zu produzieren.

10. Verfahren nach Anspruch 9, das weiterhin den Schritt des Testens des codierten Polypeptids auf gewünschte Eigenschaften aufweist.

11. Verfahren nach einem der Ansprüche 1 bis 10, wobei die Ausgangspolynukleotidsequenz einen Antikörper oder ein Fragment davon codiert.

12. Verfahren nach einem der Ansprüche 1 bis 10, wobei die Ausgangspolynukleotidsequenz ein Enzym codiert.

13. Verfahren nach einem der Ansprüche 1 bis 10, wobei die Ausgangspolynukleotidsequenz ein Antigen codiert.

14. Verfahren nach einem der Ansprüche 1 bis 13, das weiterhin den Schritt des Einbauens der mindestens einen Polynukleotidsequenz, die in dem Schritt d) erzeugt wurde, in einen Vektor aufweist.

15. Verfahren nach Anspruch 14, das weiterhin den Schritt des Exprimierens *in vitro* der mindestens einen Polynukleotidsequenz, die in einen Vektor eingebaut wurde, aufweist, um das codierte Polypeptid zu produzieren.

16. Verfahren nach Anspruch 14 oder 15, das weiterhin den Schritt des Formulierens der Polynukleotidsequenz oder von Vektoren, die das Polynukleotid aufweisen, oder des Polypeptids, das durch das Polynukleotid codiert wird, in eine pharmazeutisch akzeptable Zusammensetzung aufweist.

## Revendications

1. Procédé de génération d'une séquence de polynucléotides ou d'une population de séquences à partir de séquences polynucléotides simples brins parentes codant pour un ou plusieurs motifs protéiques, comprenant les étapes consistant à
a) fournir de l'ADN simple brin constituant les brins positifs et négatifs des séquences polynucléotides parentes ;
b) digérer les séquences polynucléotides simples brins avec une exonucléase afin de générer des populations de fragments simples brins ;
c) mettre en contact lesdits fragments générés à partir des brins positifs avec les fragments générés à partir des brins négatifs et, facultativement, ajouter des séquences d'amorce qui s'hybrident avec les extrémités 3' et 5' d'au moins un des polynucléotides parents dans des conditions d'annelage ;
d) amplifier les fragments qui s'hybrident les uns avec les autres afin de générer au moins une séquence polynucléotide codant pour un ou plusieurs motifs protéiques ayant des caractéristiques modifiées par rapport au(x) motif(s) protéique(s) codé(s) par lesdits polynucléotides parents.

2. Procédé selon la revendication 1, dans lequel l'exonucléase est sélectionnée dans le groupe constitué de BAL31, l'exonucléase I, l'exonucléase V, l'exonucléase VII, et l'exonucléase Rec J_{f}.

3. Procédé selon la revendication 2, dans lequel l'exonucléase est BAL31.

4. Procédé selon la revendication 1, 2 ou 3, dans lequel une séquence ou des séquences polynucléotide(s) parente(s) a (ont) été soumises à une mutagenèse.

5. Procédé selon la revendication 1, dans lequel la population de fragments générée à l'étape b) est soumise à une mutagenèse.

6. Procédé selon la revendication 4 ou 5, dans lequel la mutagenèse est une PCR sujette à erreur.

7. Procédé selon l'une quelconque des revendications 1 à 6, dans lequel l'étape b) est mise en oeuvre afin de générer une population de fragments simples brins de différentes longueurs.

8. Procédé selon la revendication 7, dans lequel l'étape b) est contrôlée afin de générer une population de fragments simples brins ayant une longueur moyenne de plus qu'approximativement 50 nucléotides.

9. Procédé selon l'une quelconque des revendications 1 à 8, comprenant encore l'étape d'expression d'au moins une séquence polynucléotide générée à l'étape d) afin de produire le polypeptide codé.

10. Procédé selon la revendication 9 comprenant en outre l'étape de test des caractéristiques souhaitées du polypeptide codé.

11. Procédé selon l'une quelconque des revendications 1 à 10, dans lequel la séquence polynucléotide parente code pour un anticorps ou un fragment de celui-ci.

12. Procédé selon l'une quelconque des revendications 1 à 10, dans lequel la séquence polynucléotide parente code pour une enzyme.

13. Procédé selon l'une quelconque des revendications 1 à 10, dans lequel la séquence polynucléotide parente code pour un antigène.

14. Procédé selon l'une quelconque des revendications 1 à 13 comprenant en outre l'étape d'incorporation d'au moins une séquence polynucléotide générée à l'étape d) dans un vecteur.

15. Procédé selon la revendication 14 comprenant en outre l'étape d'expression *in vitro* d'au moins une séquence polynucléotide incorporée dans un vecteur afin de produire le polypeptide codé.

16. Procédé selon la revendication 14 ou 15, comprenant en outre l'étape de formulation de la séquence polynucléotide ou de vecteurs comprenant les polynucléotides ou le polypeptide codé par les polynucléotides dans une composition pharmaceutiquement acceptable.
